# EUROPEAN PATENT APPLICATION

(11) **EP 2 025 335 A1**
(43) Date of publication of application: **18.02.2009**
(21) Application number: 07016037.9
(22) Date of filing: 16.08.2007
(51) Int. Cl.: A61K 31/085, A61K 31/215, A61K 31/343, A61K 36/79, A61K 36/57, A61P 3/04, A61P 3/06

(54) **Extracts with liver-X-receptor modulators, compounds and their use in weight control and treatment of disorders of lipid metabolism**

(71) Applicant: InterMed Discovery GmbH, 44227 Dortmund (DE)
(72) Inventor: Grothe, Torsten, Dr., 51063 Köln (DE); Roemer, Ernst, Dr., 07751 Bucha (DE); Henkel, Thomas, Dr., 42111 Wuppertal (DE)
(74) Representative: Maucher, Wolfgang

(57) **Abstract**

The invention relates to the use, or methods (especially with regard to animals, especially human, that are in need of such treatment) comprising the use, of an extract and/or one or more natural compounds from plants or parts of plants, respectively, from a genus selected from the group consisting of *Schisandra, Illicium, Kadsura, Steganotaenia* and *Magnolia,* alone or as supplement, as active ingredient in the regulation of body weight and/or fat loss and/or for the management of obesity, either in humans or in animals, to the use of said extract and/or natural compound(s) or mixtures in the manufacture of a pharmaceutical or nutraceutical formulation for the regulation of body weight and/or fat loss and/or for the management of obesity either in humans or in animals. The above extract and/or compound(s) can further be used to reduce one or more adverse metabolic parameters in a subject, such as the blood cholesterol level, especially the "bad" low density lipid (LDL) cholesterol. The invention relates also to said extract and/or compound(s) for use in the treatment or in the preparation of a medicament for the treatment of obesity and/or elevated blood cholesterol, as well as their preparation. It also relates to pharmaceutical or nutraceutical formulations comprising said extract and/or natural compound(s) which are useful in the regulation of body weight and/or fat loss and/or for the management of obesity.

## Description

### Summary of the invention

The invention relates to the use, or methods (especially with regard to animals, especially human, that are in need of such treatment) comprising the use, of an extract and/or one or more natural compounds from plants or parts of plants, respectively, from a genus selected from the group consisting of *Schisandra, Illicium, Kadsura, Steganotaenia* and *Magnolia,* alone or as supplement, as active ingredient in the regulation of body weight and/or fat loss and/or for the management of obesity, either in humans or in animals, to the use of said extract and/or natural compound(s) or mixtures in the manufacture of a pharmaceutical or nutraceutical formulation for the regulation of body weight and/or fat loss and/or for the management of obesity either in humans or in animals. The above extract and/or compound(s) can further be used to reduce one or more adverse metabolic parameters in a subject, such as the blood cholesterol level, especially the "bad" low density lipid (LDL) cholesterol. The invention relates also to said extract and/or compound(s) for use in the treatment or in the preparation of a medicament for the treatment of obesity and/or elevated blood cholesterol, as well as their preparation. It also relates to pharmaceutical or nutraceutical formulations comprising said extract and/or natural compound(s) which are useful in the regulation of body weight and/or fat loss and/or for the management of obesity.

### Background of the invention

Weight control is a concern of human beings. More importantly, excessive accumulation of body fat (i.e. obesity (= adiposity), especially with excessive fat in the ventral region and surrounding the viscera)) can be dangerous and has been linked to health problems such as type II diabetes, hypertension, heart disease, atherosclerosis (where more than two of the preceding disorders are present, the condition is often called "Metabolic Syndrome"), hyperlipidemia, coronary heart disease, stroke, breast and colon cancer, sleep apnoea, gallbladder disease, gastroesophageal reflux disease, fatty liver, gout or thromboembolism. Obesity is one of the main factors in the development of cardiovascular diseases. The levels of cholesterol, blood pressure, blood sugar and uric acid in obese people are usually higher than those of persons of normal weight. The morbidity from coronary heart disease among the overweight people is increased as well. Among the people aged 40-50, mortality will rise about 1 % when body weight increases by 0.5 kg and the death rate will increase 74% when body weight exceeds 25% of the standard. The prevalence of obesity in the United States has more than doubled since the turn of the last century (whole population) and more than tripled within the last 30 years among children aged from 6 to 11. This problem more and more becomes a disease risk also in Europe. In Germany, particularly many people have been found to suffer from overweight recently, already 25% of the young people, children and adolescents there are affected by obesity and related disorders. Furthermore, being overweight is considered by the majority of the Western population as unattractive.

Determinants of obesity include social factors, psychological factors, genetic factors, developmental factors and decreased physical activity. Some components of a comprehensive weight loss programs include medical assessment, behavioural and dietary modification, nutrition education, mental and cognitive restructuring, increased physical activity, and long term follow-up.

An increasing interest by consumers in the maintenance or reduction of their body weight can be found. This leads to a demand for products useful for these purposes. Preferred are such food products which can conveniently be consumed as part of the daily diet, for example meal replacer products, such as meal replacer bars and beverages. These are usually designed for use as a single-serving food product to replace one or two meals a day.

An issue is that often a saturating effect is missed when such products are consumed, resulting in hunger feelings only a relatively short time after consummation or even in the lack of a saturation feeling already directly after consummation.

Summing up, there remains a need for safe and effective compositions for promoting weight loss and/or loss of body fat in subjects such as humans. The problem to be solved by the present invention is therefore to find compositions or compounds useful in the treatment of obesity and/or elevated blood cholesterol levels.

Phytochemistry provides a large pool of compounds and compositions to be looked at whether they are able to solve this problem.

Plants like *Schisandra chinensis* are known from the traditional Chinese medicine (TCM) as Wu Wei Zi. The TCMs are essential components of alternative medicines. Many TCMs are known to alter the expression of hepatic drug-metabolizing enzymes and transporters. The molecular mechanisms by which TCMs and/or their constituents regulate enzyme and/or transporter expression, however, have remained largely unknown.

Plants used in medicine originate from all areas on earth. They or compounds or extracts therefrom are, for example, used in traditional medicine. Thus, Schizandrin B is a hepato-and cardioprotective ingredient isolated from the fruit of *Schisandra chinensis,* a traditional Chinese herb clinically used to treat viral and chemical hepatitis (see Chiu PY, et al., Mol. Cell. Biochem. 2004 266(1-2):139-44). Extracts from the fruits (seeds) of *Schisandra chinensis L.* and pure isolated substances are one of the components of medicinal preparations designed for the treatment of cardiovascular diseases, liver diseases, as a supplement in the treatment of neoplasms, diabetes, and the like (see Opletal L et al., Krenkova M, Havlickova P; Phytotherapeutic aspects of diseases of the circulatory system. 8. Chinese magnolia (Schisandra chinensis (Turcz.) Baill.): production of the drugs and their evaluation, therapeutic and dietary preparations. Ceska Slov. Farm. 2001 50(5):219-24).

The Dictionary of Natural Products quotes 151 different compounds for the class of dibenzo[a,c]cyclooctadiene lignans (see Dictionary of Natural Products, Version 15:1, Chapman & Hall/CRC 2007**).** Several biological activities are named, such as: inhibition of P-glycoprotein and of multidrug resistance-associated protein-1, inhibition of HIV-1 protease, and inhibitory activity on NFAT transcription (see Lee IS et al., Planta Med. 2003 69(1):63-4). Complex mixtures like plant extracts containing *Schisandra sp.* from TCM were reported to exhibit activities like: activation of the pregnane X receptor and increase of warfarin clearance in rats (see Mu Y et al., J. Pharmacol. Exp. Ther. 2006 316(3):1369-77), antibacterial activity against Salmonella (see Lee MH et al., Int. J. Food Microbiol. 2006 111 (3):270-5) or against Helicobacter pylori (see Li Y et al., J. Ethnopharmacol. 2005 98(3):329-33), endothelium-dependent and -independent relaxation of isolated rat thoracic aorta (see Rhyu MR et al., Phytomedicine. 2006 May 14; [Epub ahead of print), protection against adriamycin-induced cardiotoxicity in rats (see You JS et al., Chang Gung Med J. 2006 29(1):63-70), attenuation of the neurotoxicity induced by L-glutamate (see Kim SR et al., J. Neurosci. Res. 2004 76(3):397-405).

Moreover, there were activities reported to be related to the liver, the activity of liver enzymes, liver protecting activities and liver illnesses, such as: induction of antioxidant response in mouse liver (see Chiu PY et al., Mol. Cell. Biochem. 2006 Aug 24; [Epub ahead of print]), reversion of multidrug resistance (MDR) in Pgp-overexpressing HepG2-DR cells (see Wan CK et al., Biochem. Pharmacol. 2006 72(7):824-37), enhancement of cellular glutathione and heat shock protein production as well as protection against oxidant injury in H9c2 cardiomyocytes (see Chiu PY et al., Mol. Cell. Biochem. 2006 289(1-2):185-91), inhibitory effect on human liver microsomal erythromycin N-demethylation activity mediated by cytochrome P450 3A4 (CYP3A4) (see Iwata H et al., Drug Metab. Dispos. 2004 32(12):1351-8), antihepatitis activity on anti-HBsAg (human type B hepatitis, surface antigen) and on anti-HBeAg (human type B hepatitis, e antigen) (see Wu MD et al., Chem. Pharm. Bull. (Tokyo). 2003 51 (11):1233-6), and inhibition of the increase in number and size of GST-P positive foci, regardless of the promoter (see Miyamoto K et al., Biological & pharmaceutical bulletin, 1995 18(10):1443-5).

The CNS neuropeptide FF (NPFF) is believed to play a role in pain modulation and opiate tolerance. Its two G-protein coupled receptors, NPFF1 and NPFF2 have been identified and isolated from the human central nervous system. Recently Gomisin G and benzoylgomisin Q were identified as potent NPFF2 receptor agonists. A correlation between this activity and obesity was suggested but no data were shown to prove this pronouncement (see Do EU et al., Peptides. 2006 27(5):997-1004). Only derivatives to be synthesized are suggested as of potential interest to develop new therapeutics as potential anti-obesity drugs. However, it is not clear whether such compounds could at all reach the NPFF receptors in the central nervous system, as they may not be able to pass the blood brain barrier. In addition, no *in vivo* assays are provided in the publication to support any such treatment options. Thus this provides a disincentive to look for substances from natural sources.

WO 2004/082700 describes compositions including as basic constituent the extracts from four plants and an animal, namely *Coix lachrymajobi* (belonging to the *Poaceae*), *Castanea crenata* (belonging to the *Fagaceae*), *Cervus elaphus* (cornu of *deer*) , *Nelumbo nucifera* (belonging to the *Nympaeaceae*) and *Schizandra chinensis* (belonging to the *Magnoliaceae*) and other plant extracts (from *Disocorea batatas, Platycodon grandiflorum, Liriope platyphylla, Morus alba, Raphanus sativus, Pyrus ussuriensis, Prunus mune, Phyllostachys bambusoides* and *Angelica keiskei*) used in the treatment of obesity. Only a combination of all these 14 extracts is examined in the example and shown to reduce the weight of mice by 6.4 to 9.2 %. Also the blood lipid levels show positive effects. However, due to the high number of extracts administered, it is not possible to understand whether the removal of one of more of them would be possible without loosing the effects. For example, no data are provided even for the combination of only the five basic constituents.

WO 2004/096252 describes compositions comprising "mushroom powder, extract, or derivative thereof" (where the mushroom can be selected from the group consisting of Agaricus, Auricularia, Cordyceps, Coriolus, Ganoderma, Grifola, Hericuim, Lentinus, Pleurotus, Polyporus, Poria, Trametes, Tremella and combinations thereof) in combination with a liver protecting agent, such as one selected from the group consisting of Andrographis, artichoke, Artemisia, astragalus, barberry, boldo, bupleurum, dandelion, dong quai, fo-ti, fringe tree, fumitory, gotu kola, guggul, kudzu, licorice, lyceum, milk thistle, neem, Phyllanthus, Picorrhiza, prickly pear, rehmannia, skullcap, schisandra, tumeric and combinations thereof. Thus schisandra is only mentioned among a number of liver protecting agents and not described as responsible for the anti-obesity activity, while only the mushrooms are described as selected on the basis of a hunger suppressing activity and fat burning capability (page 9 lines 12 to 13). Thus the anti-obesity effect relies on the mushrooms. No data on body fat are presented in the Examples, and it appears that the mice compared in Fig. 4 rather show a general negative effect on growth of the mice than clear evidence of obesity treatment

The activation of the xenobiotic orphan nuclear pregnane X receptor and induction of detoxifying enzymes provide a molecular mechanism for the hepatoprotective effects of certain TCMs. Extracts of *Schisandra chinensis* and their main constituents Schizandrol and Schizandrin were found to induce the expression of drug-metabolizing enzymes and transporters in reporter gene assays and in primary hepatocyte cultures (see Mu Y et al., J. Pharmacol. Exp. Ther. 2006 316(3):1369-77).

Liver X receptors (LXR) are nuclear hormone receptors that play a critical role in cholesterol homeostasis. LXR agonists are expected to increase cholesterol efflux, lower LDL (the "bad" cholesterol) and raise HDL (the "good" cholesterol) levels (see Zelcer N et al., Curr. Opin. Investig. Drugs. 2005 6(9): 934-943, and Geyeregger R et al., Cell. Mol. Life Sci. 2006 63(5): 524-539). Known LXR agonists were discovered by screening libraries from natural sources and proof of principle in animal models was possible (see Herath KB, et al., J. Nat. Prod. 2005 68: 1437-1440 ; Jayasuriya H et al., J. Nat. Prod. 2005 68: 1247-1252 ; and Singh SB et al., Bioorg. Med. Chem. Lett. 2005 15(11): 2824-2828).

The citing of references within the present application is not intended to mean an admission that the respective references are relevant prior art.

### General description of the invention

Surprisingly, it has now been found that already extracts and/or one or more compound(s) from extracts of plants or parts of plants of the genus *Schisandra* (and the genera *Illicium, Kadsura, Steganotaenia and Magnolia*) alone have activity in the regulation of body weight and/or fat loss, especially for the management of obesity, and are capable of allowing weight control in animals and thus the treatment of obesity, as well as treatment of other conditions mentioned herein.

Thus it is possible to use such extracts and constituents therefrom, especially lignanes or mixtures thereof, alone or in combination with useful combination partners, preferably other than Gomisin G, Benzoylgomisin Q, *Coix lachrymajobi, Castanea crenata,* Cervus elaphus, *Nelumbo nucifera, Disocorea batatas, Platycodon grandiflorum, Liriope platyphylla, Morus alba, Raphanus sativus, Pyrus ussuriensis, Prunus mune, Phyllostachys bambusoides, Angelica keiskei,* and mushroom powder or an extract or a derivative of mushroom powder, more preferably also excluding chemical derivatives not present in nature of Gomisin G or Benzyolgomisin Q obtainable solely by synthetic means.

Surprisingly, enriched extracts obtained from *Schisandra chinensis* fruit as well as some purified compounds out of the extracts now can be shown to exhibit activating effect on the LXR, and especially they also show an effect inducing weight loss or decreased weight gain in mice. Even more surprisingly, these enriched extracts need not comprise Gomisin G or Benzoylgomisin Q for which a (though week) activity against NPFF was shown (see above) but which, as will be shown in the examples, are not expected to pass the blood/brain barrier, so that their effect on NPFF appears not reasonable.

### Detailed description of the invention

Accordingly, in a first embodiment, the invention relates to the USE of extracts from one or more plants or parts of one or more plants preferably from the genera *Schisandra, Illicium, Kadsura, Steganotaeinia* and *Magnolia,* especially *Schisandra,* comprising one or more compounds of the formula I or the USE of one or more compounds isolated therefrom, said compounds preferably being selected from dibenzo[a,c]cyclooctadiene derivatives of the formula I, 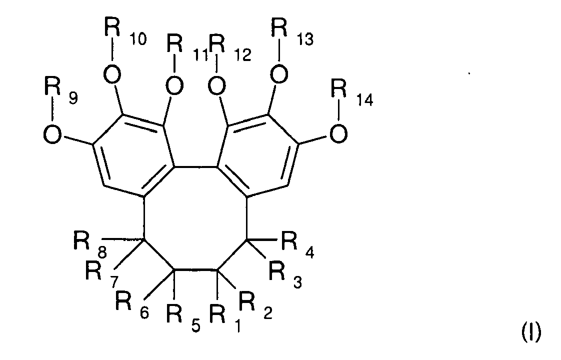 wherein
R2, R3, R4, R6, R7 and R8 independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more ring heteroatoms, a substituted or unsubstituted aryl group with 6 to 18 ring atoms which can comprise one or more ring heteroatoms, a substituted or unsubstituted C₆-C₁₈-aryl-C₁-C₁₂-alkyl group which can comprise one or more heteroatoms, a substituted or unsubstituted aryloxy group wherein aryl has 6 to 18 ring atoms and can comprise one or more ring heteroatoms, a (-C(O)-Rₐ) group, a (-C(S)-Rₐ) group, a (-OC(O)-Rₐ) group, a (-OC(S)-Rₐ) group, a (-OC(O)-ORₐ) group, a (-(CH₂)ₙ-CRₐ-CRₐR_{b}) group, a (-O-(CH₂)ₙ-CRₐ=CRₐR_{b}) group or a (-OC(O)-(CH₂)ₙ-CRₐ=CRₐR_{b}) group wherein n is zero or an integer from 1 to 12 and
Rₐ and R_{b} independently from each other are selected from a group A, consisting of hydrogen, a straight-chain or branched-chain alkyl group having 1 to 12 carbon atoms, a cycloalkyl group having 3 to 10 carbon atoms and optionally comprising one or more ring heteroatoms, and a substituted or unsubstituted aryl group with 6 to 18 ring atoms which can comprise one or more ring heteroatoms, and a substituted or unsubstituted C₆-C₁₈-aryl-C₁-C₁₂-alkyl group which can comprise one or more heteroatoms; where R_{b} can further be selected from (CH₂-CH₂-CRₐ=CRₐRₐ) with Rₐ as defined above,
R1 and R5 independently from each other represent a (CH₂Rₐ) group, a (CH₂ORₐ) group, a (-C (O)-Rₐ) group, a (-C(S)-Rₐ) group, a (-COORₐ) group or a (-C(O)NRₐ) group with Rₐ as defined above;
or R3 and R4 when taken together and/or R7 and R8 when taken together respectively stand for an oxo group (=O), thioxo group (=S), imino group (=NRₐ), cyanimino group (=NCN) or alkylidene group (=CRₐR_{b}) where Rₐ and R_{b} are as defined above, or
R3 and R4 when taken together and/or R7 and R8 when taken together, respectively, forms together with the respective spiro carbon atom of the cyclooctadiene ring a 5 to 8 membered cyclic ketal structure that is unsubstituted or substituted by one or more substituents selected from group a;
or R1 and R2 when taken together and/or R5 and R6 when taken together, respectively, together with the binding atoms form a cyclic lactone and/or lactol and/or oxirane structure of selected from those with the formulae 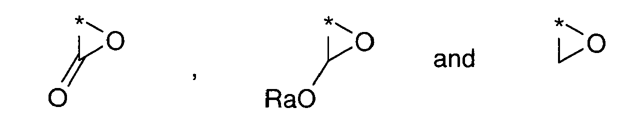 wherein the asterisk marks the carbon atom that binds R1 and R2 and/or R5 and R6 and Rₐ is as defined above,
or R1 and R3 or R4 when taken together and/or R5 and R7 or R8 when taken together, respectively, together with the binding atoms form a cyclic lactone and/or lactol and/or an oxetane structures selected from those with the formulae, 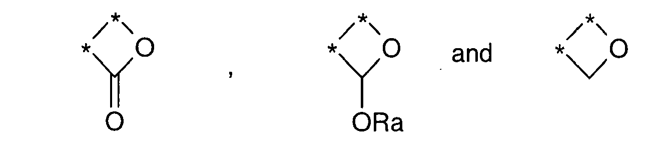 wherein the two asterisks in each formula mark the carbon atoms in formula I to which R1 and R3 or R4 and/or to which R5 and R7 or R8 are bound in formula I and Rₐ is as defined above,
or R1 and R5 when taken together and together with the binding atoms form one of the cyclic lactone, lactol, oxolane or anhydride structures of the following formulae, 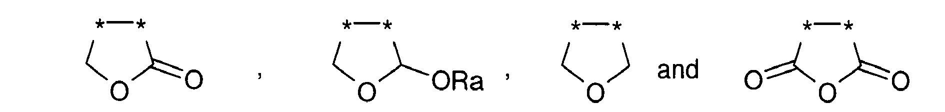 wherein the two asterisks in each formula mark the carbon atoms in formula I to which R1 and R5 are bound and Rₐ is as defined above,
or at least one of R1 and R3 or R4 when taken together and/or R5 and R7 or R8 when taken together, respectively, together with the binding atoms form a cyclic lactone and/or lactol and/or oxetane structure selected from those with the formulae, 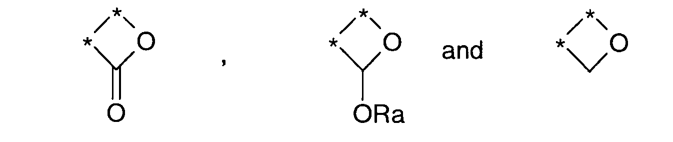 wherein the two asterisks in each formula mark the carbon atoms in formula I to which R1 and R3 or R4 and/or R5 and R7 or R8 are bound and Rₐ is as defined above,
or R1 and R7 or R8 when taken together and/or R5 and R3 or R4 when taken together, respectively, together with the binding carbon atoms and the carbon atom between them in formula I form a cyclic lactone and/or lactol and/or oxolane structure selected from those with the formulae, 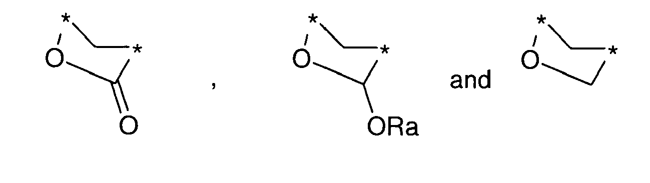 wherein the two asterisks in each formula mark the carbon atoms in formula I to which R1 and R7 or R8 and/or R5 and R3 or R4 are bound and Rₐ is as defined above,
or R1 and R6 when taken together or R5 and R2 when taken together, respectively, together with the binding carbon atoms form a cyclic lactone and/or lactol and/or oxetane structure selected from those with the formulae, 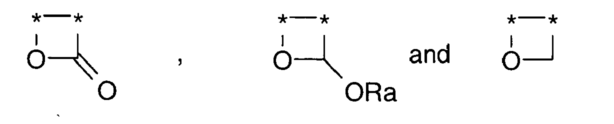 wherein the two asterisks in each formula mark the carbon atoms in formula I to which R1 and R6 or R5 and R2 are bound and wherein Rₐ is as defined above,
or R3 or R4 and R7 or R8 when taken together form oxa of the formula 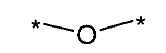 wherein the two asterisks mark the carbon atoms in formula I to which R3 or R4 and R7 or R8 are bound,
or R2 and R3 or R4 when taken together and/or R6 and R7 or R8 when taken together, respectively, together with the binding carbon atoms form a cyclic oxirane structure of the formula 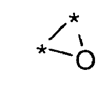 wherein the two asterisks mark the carbon atoms in formula I to which R2 and R3 or R4 and/or R6 and R7 or R8 are bound; and
R9, R10, R11, R12, R13 and R14 independently from each other represent hydrogen, a straight-chain or branched-chain alkyl group having 1 to 12 carbon atoms, a cycloalkyl group having 3 to 10 carbon atoms and optionally comprising one or more ring heteroatoms, a substituted or unsubstituted aryl group with 6 to 18 ring atoms which can comprise one or more ring heteroatoms, a substituted or unsubstituted C₆-C₁₈-aryl-C₁-C₁₂-alkyl group which can comprise one or more heteroatoms, a (-C(O)-Rₐ) group, a (-C(S)-Rₐ) group or a(-(CH₂)ₙ-CRₐ=CRₐR_{b}) group wherein n is zero or an integer from 1 to 12 and with Rₐ and R_{b} as being defined above, or
at least one of R9 and R10 when taken together, R10 and R11 when taken together, R12 and R13 when taken together and R13 and R14 when taken together, respectively, forms a straight-chain or branched-chain alkylen group having 1 to 4 carbon atoms which can comprise a heteroatom or a straight-chain or branched-chain alkenylen group having 2 to 4 carbon atoms and 1 or 2 double bonds which can comprise a heteroatom, so that together with the binding carbon atoms they form a ring;
where if one or more heteroatoms mentioned above are present they are present instead of one or more carbon atoms and are selected from the group consisting of S, N, NH, O, P and Se, preferably S, N, NH and O; with the proviso that if one or both substances with the names Gomisin G and benzoylgomisin Q are part of an extract or compound mixture useful according to the invention, at least one further compound of the formula I other than Gomisin G and benzoylgomisin Q is present, or preferably the extract or compound mixture does not comprise Gomisin G and benzoylgomisin Q.

Preferably, the compounds of the formula I are natural compounds, that is, compounds that are present in and can be isolated or extracted from natural sources (especially those mentioned in detail) without chemical synthesis steps (though they may also be prepared by chemical synthesis), and not derivatives only obtainable by chemical synthesis.

Further, the present dibenzo[a,c]cyclooctadiene derivatives of the formula I comprise all stereoisomers, such as those which may exist due to asymmetric carbons on the various substituents, including enantiomeric forms (which may exist even in the absence of asymmetric carbons, especially atropisomeres with respect to the different possible more or lass stable ring conformations of the cyclooctadiene moiety) and diastereomeric forms. Individual stereoisomers of the dibenzo[a,c]cyclooctadiene derivatives of the present invention may, for example, be substantially free of other isomers, or may be admixed, for example, as racemates or with all other, more than one other or other selected stereoisomers.

To the extent that dibenzo[a,c]cyclooctadiene derivatives of the formula I and salts thereof may exist in their tautomeric form, all such tautomeric forms are contemplated herein as part of the present invention.

Where salt-forming groups (e.g. acidic groups, such as phenolic OH groups, or basic groups, such as amino or imino groups) are present within them, the dibenzo[a,c]cyclooctadiene derivatives of the formula I may be in the free form or in the form of salts. The term "salt(s)", as employed herein, denotes acidic and/or basic salts formed with inorganic and/or organic acids and bases. In addition, when a dibenzo[a,c]cyclooctadiene derivative of the formula I contains both a basic moiety and an acidic moiety, "inner salts" may be formed and are included within the term "salt(s)" as used herein. Pharmaceutically (or nutraceutically) acceptable (i.e., non-toxic, physiologically acceptable) salts are preferred, although other salts are also useful, e.g., in isolation or purification steps which may be employed during preparation. Salts of the dibenzo[a,c]cyclooctadiene derivatives of the formula I may be formed, for example, by reacting a dibenzo[a,c]cyclooctadiene derivative of the formula I with an amount of acid or base, such as an equivalent amount, in a medium such as one in which the salt precipitates or in an aqueous medium followed by lyophilization. Salts of the dibenzo[a,c]cyclooctadiene derivatives of the formula I may also be formed by reacting a dibenzo[a,c]cyclooctadiene derivative of the formula I with an alkylating agent, for example, by quarternization of an amine, where natural compounds are preferred. Also ion exchangers can be used to form salts from free forms or free forms from salts of a dibenzo[a,c]cyclooctadiene derivative of the formula I.

Dibenzo[a,c]cyclooctadiene derivatives of the formula I which contain a basic moiety, may form salts with a variety of organic and inorganic acids. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerol-phos-phates, hemisulfates, heptanoates, hexanoates, hydrochlorides, hydrobromides, hydro-iodides, 2 hydroxyethanesul-fonates, lactates, maleates, methanesulfonates, 2-naphtalene-sulfonates, nicotinates, nitrates, oxalates, pectinates, per-sulfates, 3- phenylpropionates, phosphates, picrates, pivalates, propionates, salicylates, succinates, sulfates, sulfonates, tartrates, thiocyanates, toluenesulfonates, such as tosylates, undecanoates, and the like.

The dibenzo[a,c]cyclooctadiene derivatives of the formula I which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, N-methyl-D-glucamines, N-methyl-D-glucamides, t-butyl amines, and salts with amino acids such as arginine, lysine and the like. Also salts with salt-forming pharmaceutical and/or nutraceutical carrier materials are possible and encompassed by the invention.

Further, the dibenzo[a,c]cyclooctadiene derivatives of the formula I may be in the form of their solvates, such as hydrates, of these derivatives.

Within the present disclosure, the term "compound(s) of the formula I" is often used instead of "dibenzo[a,c]cyclooctadiene derivative(s) of the formula I".

The general expressions, within the present disclosure, preferably have the following meaning, where in each embodiment on, more than one or all more general expressions may, independently of each other, be replaced with the more specific definitions, thus forming preferred embodiments of the invention, respectively:

The names of organisms and compounds/substances containing "schisandr..." or "schizandr..." will be used in the same meaning and can be included in any of these written forms.

As "alkyl", methyl, ethyl, n-propyl, iso-propyl, n-butyl, iso-butyl, sec-butyl, tert.-butyl, n-pentyl or iso-pentyl can be especially preferred.

"Substituents" (also in the case of grammatically derived forms of this word (e.g. "substituted", e.g. in substituted aryl or the like) can especially be selected from the group consisting of C₁-C₇-alkyl, hydroxy, C₁-C₇-alkoxy, C₁-C₇-alkanoyloxy, C₁-C₇-alkoxycarbooxy, C₁-C₇-alkanesulfonyloxy, phenyl-C₁-C₇-alkoxy, amino, N-mono- or N,N-di-(C₁-C₇-alkyl, C₁-C₇-alkanoyl, C₁-C₇-alkoxycarbonyl, C₁-C₇-alkanesulfonyl and/or phenyl-C₁-C₇-alkyl)-amino, carboxy, C₁-C₇-alkoxycarbonyl, carbamoyl, N-mono- or N,N-di-(C₁-C₇-alkyl)-carbamoyl, sulfamoyl, N-mono- or N,N-di-(C₁-C₇-alkyl)-sulfamoyl and cyano. Preferably, in the case where one or more substituents are present, one or more, more preferably up to three, e.g. one or two such substituents, independently selected from the mentioned group, are present on the substituted moiety.

"Aryl" is preferably phenyl or naphthyl.

Where one or more, preferably 1 or 2, "heteroatoms" are present, they either are present instead of a ring carbon atom in the case of cyclic moieties (aryl, cycloalkyl) or instead of a chain carbon atom (in the case of acyclic moieties).

Wherever in the present disclosure a compound of the formula I, a mixture of compounds of the formula I or one or more compounds of the formula I are mentioned, this intends to include the free (enriched or (at least substantially) pure) form and/or one or more (especially pharmaceutically or nutraceutically salts (alone or together referred to as pharmaceutically acceptable salts hereinafter)) where salt-forming groups (e.g. phenolic OH-groups or amino or basic imino groups) are present, (a) solvate(s), (an) ester(s) and/or (a) tautomer(s) (where tautomerism, e.g. of the oxo/enol type, is possible), or mixtures of two or more of these specific forms. A mixture may be the result of an extraction and/or of admixing two or more compounds of the formula I.

"Obtainable" means that a product (e.g. extract or compound) may be obtained, preferably it is obtained by the specified method.

Where ratios of components are given in %, this means weight %, if not indicated otherwise.

By the term "extract", either a direct extract (in liquid or preferably dried form), e.g. obtained as described below, or preferably a further enriched extract (obtainable e.g. by one or more further purification steps after extraction, e.g. chromatography, for example as described below) containing one or more, preferably two or more compounds of the formula I is meant.

Preferably, the total weight share of the compound or compounds of the formula I in an extract or mixture of compounds of the formula I or a purified compound of the formula I that is of USE according to the invention in the final extract, mixture or compound (direct or further enriched) is in the range from 0,01 to 100 % by weight, more preferably from 0,02 to 95 %, most preferably 0,05 to 95 %, from 0.05 to 50 % or e.g. from 0.1 to 90 %.

"One or more compounds of the formula I" or "a mixture of compounds of the formula I" means that either only one compound (in substantially pure form or as a direct extract or a further enriched extract) or a mixture of two or more compounds of the formula I (which mixture is preferred) can be present in an extract or pharmaceutical/nutraceutical formulation according to the invention or be of USE according to the invention.

By the term "regulation of body weight and/or fat loss" and/or "management of obesity" (especially leading to weight loss, that is, preferably use for weight/fat reduction), there is especially meant that by administration of one or more compounds of the formula I or preferably an (especially further enriched) extract comprising one or preferably two or more compounds of the formula I either a lower weight gain can be observed with the same offered diet, e.g. when compared with a control without such administration, or preferably a weight loss can be observed. More preferably, the weight loss is due to a reduction of the body fat. Thus, most preferably, the treatment of obesity is meant, either prophylactically to avoid a weight gain or preferably to reduce the body weight, especially to reduce the body fat. The term "prophylactically", in addition to treatment if a risk is present, also implies a generally healthy nutrition with an effect also in the healthy organism, e.g. in relation to elder people, e.g. in the sense of a maintenance of an appropriate, especially low, Body Mass Index (BMI).

For testing, it is possible to conduct clinical trials (or animal assays as described in the Examples). e.g. clinical trials with humans (or other animals) analogous to those described in WO 2004/096252 or WO 2004/082700 (which are incorporated here by reference, especially with regard to the description of the tests on animals or especially humans), but only using one or more compounds of the formula I or an extract comprising such compound(s) of the formula I as described for the present invention.

The extracts or compounds according to the invention may be used as such, in the form or pharmaceutical or nutraceutical formulations (the latter term including food additives) or in the form of functional food.

Where the compounds or mixture of compounds of the formula I, especially extracts comprising one or more compounds of the formula I, are used as supplement, this means that the compound(s), extract or a pharmaceutical or nutraceutical formulation comprising it or them can be added to any other nutrient or pharmaceutical or nutraceutical, preferably other than (exclude especially mixtures known). Thus they can especially serve as food supplement. However, the compound(s), extract or formulations may also be administered as such. Preferably, the supplement is not to be combined with Gomisin G, Benzoylgomisin Q, *Coix lachrymajobi, Castanea crenata,* Cervus elaphus, *Nelumbo nucifera, Disocorea batatas, Platycodon grandiflorum, Liriope platyphylla, Morus alba, Raphanus sativus, Pyrus ussuriensis, Prunus mune, Phyllostachys bambusoides, Angelica keiskei,* and mushroom powder or an extract or a derivative of mushroom powder, or more preferably also not with chemical derivatives not present in nature of Gomisin G or Benzoylgomisin G obtainable solely by synthetic means.

The activity against obesity can, for example, be tested as described in the Examples.

"Nutraceuticals", "Functional Food", or "Functional Food products" (sometimes also called "Foodsceuticals", "Medicinal Food" or "Designer Food") for USE according to the present invention are defined as food products (including beverages) suitable for human consumption - the expression comprises any fresh or processed food having a health-promoting and/or disease-preventing property beyond the basic nutritional function of supplying nutrients, including food made from functional food ingredients or fortified with health-promoting additives, especially with effects in the prophylaxis or treatment of obesity, especially allowing for body weight reduction and/or body weight maintenance, appetite suppression, the provision of satiety or other changes in metabolism, e.g. based on LXR agonism or other modulatory activities, especially outside the blood/brain barrier, and in which an extract, compound or compound mixture of compounds of formula I, respectively, according to the invention is used as an ingredient (especially additive) as health benefit agent, especially in an effective amount.

"Comprising" or "including" or "having" wherever used herein is meant not to be limiting to any elements stated subsequently to such term but rather to encompass one or more further elements not specifically mentioned with or without functional importance, that is, the listed steps, elements or options need not be exhaustive. In contrast, "containing" would be used where the elements are limited to those specifically after "containing".

Where "about" is used or a specific numerical value is given without explicitly mentioning "about", this preferably means that a given value may deviate to a certain extent from the value given, e.g. preferably by ± 20 % of the given numerical value, more preferably by ± 10 %.

The functional food products or pharmaceutical products may be manufactured according to any suitable process, preferably comprising extraction of one or more compounds of the formula I and admixing to a functional food product or at least one nutraceutically or pharmaceutically acceptable carrier.

Preferably, a functional food or a pharmaceutical or nutraceutical formulation comprising a compound, more preferably a compound mixture, for USE according to the present invention, can be obtained by
(a) extraction of one or more compounds and/or mixture of compounds of the formula I from one or more plants of the genera *Illicium, Kadsura, Steganotaenia, Magnolia and especially Schisandra,* more especially *Schisandra chinensis*;, and
(b) mixing the resulting one or more compounds and/or mixtures of compounds as active ingredient in the preparation of the functional food product with the other constituents thereof or in order to obtain a pharmaceutical or nutraceutical formulation with one or more carrier materials or with a solvent, e.g. water or an aqueous solvent (e.g. to give a juice or dispersion or solution).

Further processing steps may precede and/or follow, such as drying (e.g. freeze-drying, spray-drying and evaporation), granulation, agglomeration, concentrating (e.g. to syrups, formed via concentration and/or with the aid of thickeners), pasteurizing, sterilizing, freezing, dissolving, dispersing, filtering, centrifuging, confectioning, and the like.

When one or more compounds and/or a compound mixture according to the invention are added to a food product or pharmaceutical or nutraceutical, this also results in a functional food product or pharmaceutical or nutraceutical formulation according to the invention.

Preferably, a functional food product according to the invention comprises 0,01 to 30, e.g. 0,02 to 20, such as preferably 0.05 to 5, weight-% of a compound or mixture of compounds of the formula I or of an (especially further enriched) extract according to the invention, the rest being food and/or nutraceutically acceptable carriers and/or customary additives. Further additives may be included, such as vitamins, minerals, e.g. in the form of mineral salts, unsaturated fatty acids or oils or fats comprising them, other extracts, or the like.

The functional food products according to the invention may be of any food type. They may comprise one or more common food ingredients in addition to the food product, such as flavours, fragrances, sugars, fruit, minerals, vitamins, stabilisers, thickeners, dietary fibers, protein, amino acids or the like in appropriate amounts, or mixtures of two or more thereof, in accordance with the desired type of food product. Preferably, they do not comprise any one or more of the following constituents: Gomisin G, Benzoylgomisin Q, *Coix lachrymajobi, Castanea crenata,* Cervus elaphus, *Nelumbo nucifera, Disocorea batatas, Platycodon grandiflorum, Liriope platyphylla, Morus alba, Raphanus sativus, Pyrus ussuriensis, Prunus mune, Phyllostachys bambusoides, Angelica keiskei,* mushroom powder or an extract or a derivative of mushroom powder, or a chemical derivative not present in nature of Gomisin G or Benzyolgomisin Q obtainable solely by synthetic means. "Not comprise" means that with the test systems mentioned in the examples the compounds can not be found at all or, to be on the safe side, in amounts of not more than 0.8 % by weight, more preferably not more than 0.4 %, yet more preferably not more than 0.1 %, still more preferably 0.05 % by weight of the total peak area in HPLC of an extract or mixture of compounds of the formula I as useful according to the invention, that is, if at all then only in amounts not to be expected to be pharmacologically active.

Examples of basic food products and thus of functional food products according to the invention are fruit or juice products, such as orange and grapefruit, tropical fruits, banana, apple, peach, blackberry, cranberry, plum, prune, apricot, cherry, peer, strawberry, marionberry, black currant, red currant, tomato, vegetable, e.g. carrot, or blueberry juice, soy-based beverages, or concentrates thereof, respectively; lemonades; extracts, e.g. coffee, tea, green tea; dairy type products, such as milk, dairy spreads, quark, cheese, cream cheese, custards, puddings, mousses, milk type drinks and yoghurt; frozen confectionary products, such as ice-cream, frozen yoghurt, sorbet, ice milk, frozen custard, water-ices, granitas and frozen fruit purees; baked goods, such as bread, cakes, biscuits, cookies or crackers; spreads, e.g. margarine, butter, peanut butter honey; snacks, e.g. chocolate bars, muesli bars; pasta products or other cereal products, such as muesli; ready-to-serve-dishes; frozen food; tinned food; syrups; oils, such as salad oil; sauces, such as salad dressings, mayonnaise; fillings; dips; chewing gums; sherbet; spices; cooking salt; instant drink powders, such as instant coffee, instant tee or instant cocoa powder; instant powders e.g. for pudding or other desserts; or the like.

One or more other customary additives may be present, such as flavour, fragrances or other additives, such as one or more selected from stabilizers, e.g. thickeners; colouring agents, such as edible pigments or food dyes; bulking agents, such as fruit pulp, e.g. in dried form; polyols, such as xylitol, mannitol, maltitol or the like; preservatives, such as sodium or potassium benzoate, sodium or calcium carbonate or other food grade preservatives; antioxidants, such as ascorbic acid, carotionoids, tocopherols or polyphenols; mono-, oligo-or polysaccharides, such as glucose, fructose, sucrose, soy-oligosaccharides, xylo-oligosaccharides, galacto-oligosacharides; other artificial or natural non- or low-caloric sweeteners, such as aspartame or acesulfame; bitterness blockers; acidifiers in the form of edible acids, such as citric acids, acetic acid, lactic acid, adipic acid; flavours, e.g. artificial or natural (e.g. botanical flavours); emulsifiers; thiols, e.g. allylic thiols; diluents, e.g. maltodextrose; wetting agents, e.g. glycerol; stabilizers; coatings; isotonic agents; absorption promoting or delaying agents; and/or the like.

The one or more compounds of the formula I or compound mixtures thereof according to the invention can also be comprised in confectioned formulations to be added to foods including beverages, e.g. in the form of powders or granules, e.g. freeze-dried or spray-dried, concentrates, solutions, dispersions or other instant form, or the like.

The pharmaceutical or nutraceutical formulation (= compositions) according to the present invention can be prepared in various forms, such as granules, tablets, pills, syrups, solutions, dispersions, suppositories, capsules, suspensions, salves, lotions and the like. Pharmaceutical grade or food grade organic or inorganic carriers and/or diluents suitable for oral and topical use can be used to formulate compositions containing the therapeutically-active compounds. Diluents known in the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The compositions may also include one or more of the following: carrier proteins such as serum albumin; buffers; fillers such as microcrystalline cellulose, lactose, corn and other starches; binding agents; sweeteners and other flavouring or fragrancing agents; coloring agents; and polyethylene glycol. Those additives are well known in the art, and are used in a variety of formulations.

By "administered" herein is meant administration of a prophylactically and/or therapeutically effective dose of a dibenzo[a,c]cyclooctadiene derivative of the formula I or a mixture of compounds of the formula I, or an extract comprising one or more of them, to an animal, especially a patient. By "therapeutically effective dose" herein is meant a dose that produces the effects for which it is administered, especially a reduction of weight, more especially due to body fat reduction.

An animal or human, especially being a "patient" or "subject" for the purposes of the present invention, includes especially humans and further other (especially warm-blooded) animals. Thus, the dibenzo[a,c]cyclooctadiene derivative(s) of the formula I or a mixture of compounds of the formula I, or an extract comprising one or more of them, are applicable to both humans and animals. In the preferred embodiment the patient is a human. The patients will be treated either in prophylactic or therapeutic intention, the latter e.g. to avoid regain in weight after a weight (especially body fat) reduction (e.g. to avoid the yo-yo effect), or to avoid weight gain (especially due to an increase in body fat) *ab initio.*

Typically, the dibenzo[a,c]cyclooctadiene derivative(s) of the formula I or a mixture of compounds of the formula I, or an extract comprising one or more of them, having therapeutical activity mentioned hereinbefore (e.g. weight control, weight loss, body fat reduction, or also agonistic activity on the liver X receptor, decrease of blood fat level, decrease of cholesterol level) may be administered with at least one physiologically (= pharmaceutically or nutraceutically) acceptable carrier to a patient, as described herein. The total concentration of therapeutically active dibenzo[a,c]cyclooctadiene derivative(s) of the formula I or a mixture of compounds of the formula I or extracts comprising them in the formulation may vary from about 0.001-100 wt %, e.g. from 0,1 to 50 % by weight, the rest being the carrier material(s) and/or customary additives.

The dibenzo[a,c]cyclooctadiene derivatives of the formula I or a mixture of compounds of the formula I or extracts comprising them may be administered alone or in combination with other treatments, i.e., other anti-obesity agents, common diets or the like.

Combination does not necessarily mean a fixed combination but may also mean that the compound(s) of the formula I or the extract comprising it or them may be administered in a chronically staggered manner with the combination partner(s), e.g. in the form of a kit of parts (which also is an embodiment of the invention) with other combination partners, other than those excluded hereinbefore. Preferably, the chronically staggered administration takes place such that the combination partners mutually influence, especially intensify (e.g. by way of an additive or preferably synergistic effect) their therapeutic efficiency.

Among other anti-obesity agents that may be combined, antilipidemics, e.g. atorvastatin, cerivastatin, fluvastatin, lovastatin, pravastatin, rosuvastatin, simvastatin, anti-obesity drugs, such as suppressants of the appetite, stimulators of the body's metabolism, or drugs or compositions interfering with the body's ability to absorb specific nutrients, such as sibutramine, diethylpropion, phendimetrazine, phentermine, fenfluramine, sibutramine, lipase inhibitors, such as orlistat; anorectics, such as dexedrine; cannabinoid receptor antagonists, such as rimonabant; acarbose; or the like, can be mentioned. Other helpful drugs or active agents may be administered, e.g. psychoactive agents, agents that help in the treatment of addictive behaviour, e.g. nicotine addiction, or the like, especially in so far as they help to support the prophylaxis or treatment according to the invention intended.

Weight loss diets, such as food combining, Hay diet, Atkins diet (low-carbohydrate diet), cabbage soup diet, diabetic diet, fat resistance diet, slimming world diet, low-fat diet, Pritkin diet, low-carbohydrate diet, low protein diet, negative calorie diet, raw food diet, weight watchers diet are possible examples of appropriate diets.

The dibenzo[a,c]cyclooctadiene derivatives of the formula I or a mixture of compounds of the formula I or an extract comprising them, itself or as mixtures of certain complexity, eg. extracts or preparations, e.g. juices etc of the above mentioned plants, of this invention are particular useful for controlling the body weight, preferably the treatment of obesity, adipositas, and/or in regulation of lipid disorders, preferably in decreasing blood cholesterol levels, especially LDL blood cholesterol.

Natural compounds of the formula I, or extracts comprising one or more thereof, for USE according to the present invention are isolated from one or more plants of the genera *Schisandra, Illicium, Kadsura, Steganotaenia* and *Magnolia,* preferred from *Schisandra bicolor, Schisandra bicolor var. tuberculata, Schisandra chinensis, Schisandra glabra, Schisandra glaucescens, Schisandra grandiflora, Schisandra henryi, Schisandra lancifolia, Schisandra nigra, Schisandra plena, Schisandra propinqua, Schisandra propinqua var. sinensis, Schisandra pubescens, Schisandra rubriflora, Schisandra sphenanthera, Schisandra viridis, Illicium angustisepalum, Illicium anisatum, Illicium arborescens, Illicium difengpium, Illicium dunnianum, Illicium fargesii, Illicium floridanum, Illicium henryi, Illicium lanceolatum, Illicium majus, Illicium micranthum, Illicium oligandrum, Illicium parviflorum, Illicium parvifolium, Illicium spathulatum, Illicium verum, Kadsura ananosma, Kadsura angustifolia, Kadsura coccinea, Kadsura heteroclite, Kadsura induta, Kadsura japonica, Kadsura Iongipedunculata, Kadsura oblongifolia, Kadsura polysperma, Kadsura scandens, Steganotaenia araliacea, Magnolia acuminate, Magnolia acuminata var. acuminate, Magnolia acuminata var. subcordata, Magnolia albosericea, Magnolia amoena, Magnolia ashei, Magnolia biondii, Magnolia campbellii, Magnolia caricifragrans, Magnolia carsonii, Magnolia cathcartii, Magnolia championii, Magnolia coco, Magnolia cylindrical, Magnolia dawsoniana, Magnolia dealbata, Magnolia delavayi, Magnolia denudate, Magnolia dodecapetala, Magnolia elegans, Magnolia fraseri, Magnolia fraseri var. fraseri, Magnolia gigantifolia, Magnolia glabra, Magnolia globosa, Magnolia grandiflora, Magnolia griffithii, Magnolia guangnanensis, Magnolia guatemalensis, Magnolia gustavii, Magnolia henryi, Magnolia hypoleuca, Magnolia iltisiana, Magnolia kachirachirai, Magnolia kobus, Magnolia latahensis, Magnolia lenticellatum, Magnolia liliifera, Magnolia liliifera var. obovata, Magnolia macrophylla, Magnolia macrophylla subsp. macrophylla, Magnolia mahechae, Magnolia menglunensis, Magnolia mexicana, Magnolia minor, Magnolia nitida, Magnolia nitida var. lotungensis, Magnolia nitida var. nitida, Magnolia obovata, Magnolia odoratissima, Magnolia officinalis, Magnolia officinalis var. biloba, Magnolia ovata, Magnolia pacifica, Magnolia pacifica subsp. tarahumara, Magnolia paenetalauma, Magnolia panamensis, Magnolia pealiana, Magnolia phanerophlebia, Magnolia pilocarpa, Magnolia poasana, Magnolia portoricensis, Magnolia praecocissima, Magnolia pseudokobus, Magnolia pterocarpa, Magnolia pyramidata, Magnolia quinquepeta, Magnolia rostrata, Magnolia salicifolia, Magnolia sargentiana, Magnolia schiedeana, Magnolia shangsiensis, Magnolia sharpie, Magnolia sieboldii, Magnolia sieboldii subsp. japonica, Magnolia sieboldii subsp. sieboldii, Magnolia sieboldii subsp. sinensis, Magnolia sinica, Magnolia splendens, Magnolia sprengeri, Magnolia stellata, Magnolia tamaulipana, Magnolia tripetala, Magnolia virginiana, Magnolia wilsonii, Magnolia x soulangeana, Magnolia yoroconte,and*/*or Magnolia zenii,* and or from parts thereof.

Preferred extracts are obtained from *Schisandra chinensis* fam. Schisandraceae (also written as Schizandra) included the identical plants with the synonyms: *Maximowicza chinensis, Sphaerostema japonica, Kadsura chinensis* or ethnobotanic names like Wu Wei Zi, Tyosen-Gomisi, five-flavor-fruit, magnolia-vine or simply Schisandra.

Plant parts are, e.g., leaves, bark, flowers, buds, fruits, stems, shoots, roots, tubers or other parts of plants, and they or the plants can be complete, hackled, crushed, chopped up, broken up, homogenized, dried, fermented or treated otherwise.

The dibenzo[a,c]cyclooctadiene derivatives of the formula I or a mixture of compounds of the formula I , or an extract comprising one or more of them, of the present invention can be prepared by extracting and preferably enriching up to isolating them from the plants or parts of plants. Auxiliary means such as (especially ultrasonic) sonication, heating (e.g. to temperatures from room temperature to 50 °C), stirring, re-extraction, evaporation or the like, may be used to allow for appropriate extraction.

Extraction preferably takes place with a non polar or more preferably a polar solvent or solvent mixture, e.g. water and/or an alcohol, such as ethanol

Preferably, the extracts can subsequently be further enriched by one or more additional purification steps, such as distribution, precipitation (e.g. crystallisation) or especially chromatography, by which it is possible to obtain further enriched extracts or isolated compounds of the formula I.

The dibenzo[a,c]cyclooctadiene derivatives of the formula I can e.g. be isolated or the extracts prepared as described in the appended examples. The method for detection can comprise high pressure liquid chromatography (HPLC) on reversed phase silica gel (C18) with water/ acetonitrile-gradient as an elution solvent with UV as well as MS detection which are used for the product analysis and production optimization. It will be clear to those having ordinary skill in this art that the dibenzo[a,c]cyclooctadiene derivatives of the formula I, though per se natural products, can alternatively be synthesized according to standard methods leading to compounds identical with the natural compounds, where appropriate methods, for example, can be deduced from the following publications: March's Advanced Organic Chemistry: Reaction, Mechanisms and Structure, 5th ed. by Michael B. Smith, Jerry March, Wiley-Interscience; 2001; Classics in Total Synthesis: Targets, Strategies, Methods by K. C. Nicolaou, E. J. Sorensen John Wiley & Son Ltd, 1996 and The Art and Science of Total Synthesis at the Dawn of the Twenty-First Century. Nicolaou KC et al., Angew Chem Int Ed Engl 2000, 39 (1): 44 -122.

Where USE is mentioned, this especially refers to one or more of the following embodiments of the invention which can be inserted wherever USE is mentioned:
(1) A compound of the formula I, or a mixture of compounds of the formula I, or especially a (preferably further enriched) extract comprising one or more compounds of the formula I, for use in therapeutic (including prophylactic) treatment of an animal, preferably a mammal, especially a human, for the regulation of body weight and/or fat loss and/or for the management of obesity, especially for decreasing the body weight, more especially for decreasing the body fat; and/or for decreasing blood cholesterol, especially LDL cholesterol; or simply for maintenance of a healthy body, e.g. a low BMI.
(2) A pharmaceutical or nutraceutical composition comprising a compound of the formula I, or a mixture of compounds of the formula I, or especially a (preferably further enriched) extract comprising one or more compounds of the formula I, as active ingredient together with a pharmaceutically acceptable diluent or carrier, especially for use in the therapeutic and/or prophylactic treatment mentioned under (1).
(2') A pharmaceutical or nutraceutical composition for the treatment as mentioned under (1) comprising a compound of the formula I, or a mixture of compounds of the formula I, or especially a (preferably further enriched) extract comprising one or more compounds of the formula I, and a pharmaceutically acceptable diluent or carrier, as active ingredient supplement to a food.
(3) A functional food comprising a compound of the formula I, or a mixture of compounds of the formula I, or especially a (preferably further enriched) extract, as active ingredient for the treatment as mentioned under (1).
(4) A method for the treatment as mentioned under (1), especially any one or more of obesity, excess body fat and/or increased (especially LDL) blood cholesterol, in a subject in need of such treatment, comprising administering a pharmaceutically or nutraceutically effective amount of a compound of the formula I, a mixture of compounds of the formula I, or a (preferably further enriched) extract comprising one or more compounds of the formula I, as active ingredient, especially to an individual in need thereof.
(5) The use of a compound of the formula I, or a mixture of compounds of the formula I, or a (preferably further enriched) extract comprising one or more compounds of the formula I, as active ingredient for the manufacture of a medicament or nutraceutical or food supplement for the treatment mentioned under (1).
(6) A method or use as defined under (4), comprising co-administration, e.g. concomitantly or in sequence, of a therapeutically effective amount of compound of the formula I, or a mixture of compounds of the formula I, or a (preferably further enriched) extract comprising one or more compounds of the formula I, as active ingredient and a different pharmaceutically active compound and/or a pharmaceutically acceptable salt thereof, said different pharmaceutically active compound and/or salt thereof being especially for use in the treatment as mentioned under (1).
(7) A combination product comprising a therapeutically effective amount of a compound of the formula I, or a mixture of compounds of the formula I, or a (preferably further enriched) extract comprising one or more compounds of the formula I, as active ingredient, and a different pharmaceutically active compound and/or a pharmaceutically acceptable salt thereof, said second pharmaceutically active compound being especially for use or of use in the treatment mentioned under (1).

For any of the USEs, the USE is such that the compound(s) of formula I or the extract comprising such compound(s) of the formula I are the active ingredient, that is, they are already alone capable of achieving the intended effect (regulation of body weight and/or fat loss and/or management of obesity, especially decreasing of body weight, more especially decreasing body fat, and/or in the treatment of elevated blood cholesterol, especially for lowering plasma (especially LDL) cholesterol, and are thus themselves the important active principle for the treatment(s) mentioned. Throughout the present specification, the prophylactic and/or therapeutic treatment or regulation of body weight and/or fat loss and/or management of obesity, especially decreasing of body weight, more especially decreasing body fat, are especially preferred embodiments according to the invention.

In all embodiments mentioned, the USE in combination with Gomisin G, Benzoylgomisin Q, *Coix lachrymajobi, Castanea crenata,* Cervus elaphus, *Nelumbo nucifera, Disocorea batatas, Platycodon grandiflorum, Liriope platyphylla, Morus alba, Raphanus sativus, Pyrus ussuriensis, Prunus mune, Phyllostachys bambusoides, Angelica keiskei,* and mushroom powder or an extract or a derivative of mushroom powder, more preferably also excluding chemical derivatives not present in nature of Gomisin G or Benzoylgomisin Q obtainable solely by synthetic means, is preferably excluded from the scope of the invention, though the prior art did not mention that these components are actually as such active in achieving the treatment effects of the present invention (especially a USE according to the invention).

By "administering" herein is especially meant administration of a therapeutically effective dose of a compound of the formula I, or a mixture of compounds of the formula I, to a cell either in cell culture or especially to an animal, especially a human patient. By "therapeutically effective dose" herein is preferably meant a dose that produces the effects for which it is administered.

The pharmaceutical or nutraceutical preparations may be sterilized and/or may contain carrier materials or adjuvants such as preservatives, stabilizers, binders, disintegrants, wetting agents, skin or mucuous membrane penetration enhancers, emulsifiers, salts for varying the osmotic pressure and/or buffers, or other ingredients, excipients or carrier materials known in the art.

### Description of the Figures

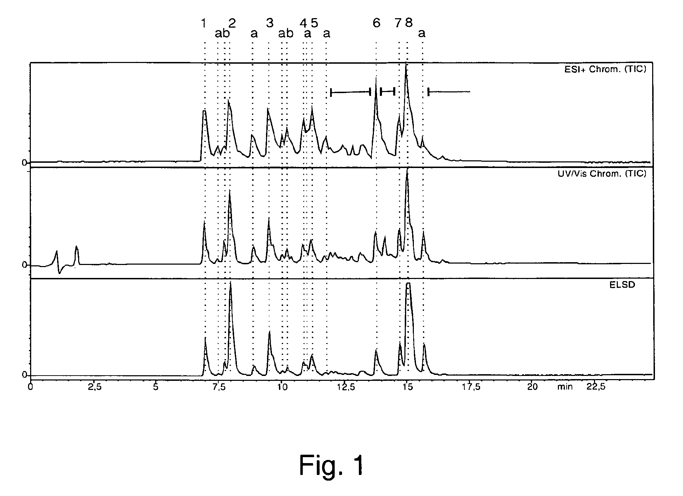

Fig. 1 shows the results of HPLC analysis of an enriched extract from *Schisandra chinensis* obtained as described in Example 2 and analyzed as described therein.
   The upper line (ESI + Chrom. (TIC)) shows the peaks obtained with Electrospray lonisation Mass Spectrometry detection as total ion count, without any selection or preferation, within the assigned range of detectable ions.
   The intermediate line (UV/Vis Chrom (TIC) shows the peaks obtained with an UV Diode Array Detector as summation of all signals within the assigned rang of wavelengths.
   The lowest line (ELSD) shows the peaks obtained with Evaporation Light Scattering Detection.

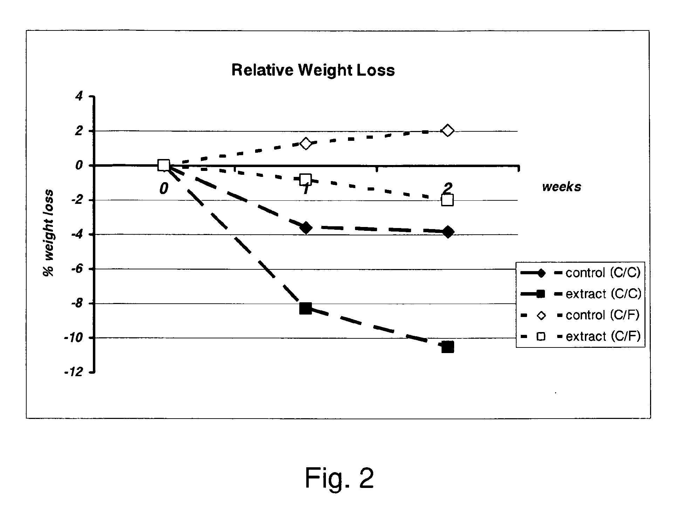

Fig. 2 shows the results from weight determinations as also represented numerically in Table 6 (see Example 2). In all cases, a weight loss can be observed with an extract according to Example 2, while the controls without administration of the extract show essentially constant weight (in general a higher weight than the treated mice).

### Preferred embodiments of the invention

The invention preferably relates to the USE of a compound or the formula I, a mixture of compounds of the formula I or preferably an (especially further enriched) extract comprising one or (preferably) two or more compounds of the formula I, wherein the compound(s) of the formula I are selected from those wherein
R1, R2, R3, R4, R5, R6, R7 and R8 are, independently of each other, hydrogen, a straight chain or branched alkyl with 1 to 5 carbon atoms, hydroxy or (-OC(O)-(CH₂)ₙ-CRₐ=CRₐR_{b}) wherein Rₐ and R_{b} are independently of each other alkyl with 1 to 5 carbon atoms, and
R9, R10, R11, R12, R13 and R14 independently from each other represent hydrogen, a straight-chain or branched-chain alkyl group having 1 to 5 carbon atoms, a cycloalkyl group having 5 to 10 ring atoms which can comprise one or more heteroatoms, a substituted or unsubstituted aryl group with 6 to 10 ring atoms which can comprise one or more heteroatoms, a substituted or unsubstituted aralkyl group which can comprise one or more heteroatoms, a (-C(O)-Rₐ) group, a (-C(S)-Rₐ) group or a(-(CH₂)ₙ-CRₐ=CRₐR_{b}) group wherein n is zero or an integer from 1 to 12 and with Rₐ and R_{b} as being defined above, or
at least one of R9 and R10 when taken together and R13 and R14 when taken together, respectively, forms a ring containing a straight-chain or branched-chain alkylen group having 1 to 4 carbon atoms which can comprise a heteroatom or a straight-chain or branched-chain alkenylen group having 2 to 4 carbon atoms and 1 or 2 double bonds,
where the heteroatoms if present are independently selected from O, S, N and NH; and where "substituted" means that one or more, especially up to three, substituents independently selected from the group consisting of C₁-C₇-alkyl, hydroxy, C₁-C₇-alkoxy, C₁-C₇-alkanoyloxy, C₁-C₇-alkoxycarbooxy, C₁-C₇-alkanesulfonyloxy, phenyl-C₁-C₇-alkoxy, amino, N-mono- or N,N-di-(C₁-C₇-alkyl, C₁-C₇-alkanoyl, C₁-C₇-alkoxycarbonyl, C₁-C₇-alkanesulfonyl and/or phenyl-C₁-C₇-alkyl)-amino, carboxy, C₁-C₇-alkoxycarbonyl, carbamoyl, N-mono- or N,N-di-(C₁-C₇-alkyl)-carbamoyl, sulfamoyl, N-mono- or N,N-di-(C₁-C₇-alkyl)-sulfamoyl and cyano are present.

More preferably the invention relates to the USE of one or more compounds of the formula I, a mixture of compounds of the formula I or preferably an (especially further enriched) extract comprising one or (preferably) two or more compounds of the formula I, wherein the compound(s) of the formula I are selected from the group consisting of those with the following names:

| systematic name | Synonyms | poss. ethnobotanic name |
|---|---|---|
| 8-Hydroxy-3,3',4,4',5,5'-hexamethoxy-2,2'-cyclolignan | **Schisandrol A** Schizandrol A Schizandrin Schisandrin | Wuweizichun A Wuweizi alcohol A |
| 3,3',4,4',5,5'-Hexamethoxy-2,2'-cyclolignan | **Desoxyschisandrol A,** Desoxyschizandrol A Desoxyschisandrin Desoxyschzandrin Schisandrin A Schizandrin A Dimethylgomisin J | Wuweizisu A |
| 3,3'-Dimethoxy-4,5:4',5'- | **Schisandrin C** | Wuweizisu C |
| bis(methylenedioxy)-2,2'-cyclolignan | Schizandrin C | |
| 7,8-Dihydroxy-3,3',4,5-tetramethoxy-4',5'-methylenedioxy-2,2'-cyclolignan-7-yl angelic acid ester | **Gomisin B** Schizantherin B Schisantherin B 7-Angeloyl Gomisin P | Wuweizi ester B |
| 7,8-Dihydroxy-3,3',4,5-tetramethoxy-4',5'-methylenedioxy-2,2'-cyclolignan-7yl benzoic acid ester | **Gomisin C** Schizantherin A Schisantherin A 7-Benzoyl Gomisin P | Wuweizi ester A |
| 3,3,4,5-Tetramethoxy-4',5'-methylenedioxy-2,2'-cyclolignan | **Gomisin N** | |
| 3,3',4,5-Tetramethoxy-4',5'-methylenedioxy-2,2'-cyclolignan-8-ol | **Gomisin A** Schisandrol B Schizandrol B Besigomsin | Wuweizisu B Wuweizichum B Wuweizi alcohol B |
| 7,8-Dihydroxy-3,3',4,5-tetramethoxy-4',5'-methylenedioxy-2,2'-cyclolignan-7-yl tiglic acid ester | **7-Tigloyl-gomisin P** | |
| 3'-O-Desmehtyl-3'-O-angeloyl-schisandrin | **Angeloylgomisin H** | |

The bold marked names in the table are used as to define the compounds Schizandrol A, Gomisin B, Gomisin C, Schisandrin C, Deoxyschizandrol A, Gomisin N, Gomisin A, 7-Tigloyl-gomisin P and Angeloylgomisin H in the following text.

Yet more preferably, in the extracts, compounds or compound mixtures, the total amount (purity) in percent by weight of the one or more compounds of the formula I in a USE according to the invention, related to impurities or in the case of an extract to other extract components, is in the range between 5 and 100 %, more preferably between 10 and 99.5 %.

Highly preferably, the invention relates to the USE of an extract obtainable in accordance with Example 1 ("crude extract") or more preferably in accordance with the enrichment in Example 2 ("further enriched extract"), preferably showing an HPLC pattern essentially similar (that is with a variance in peak height of ± 25 %, more preferably ± 15 %) to that shown in Fig. 1 according to 1, preferably 2, more preferably all three lines/methods of analysis shown there, more especially an extract (or further a compound of the formula I or preferably a mixture of compounds of the formula I) comprising any one or more compounds selected from Schizandrol A and/or especially from Schizandrol A, Gomisin B, Gomisin C, Schisandrin C, Deoxyschizandrol A, Gomisin N, Gomisin A, 7-Tigloyl-gomisin P and Angeloylgomisin H.

The invention also relates to the USE according to the invention of any one of the compounds of the formula I mentioned in the examples, alone or in combination with one or more other compounds of the formula I mentioned therein.

Finally, the invention also relates to the embodiments in the claims which are incorporated here by reference.

### Examples

The present invention is further explained by the following examples. The specific examples which follow illustrate the methods in which the compositions of the present invention may be prepared, components therein and their use, as well as other embodiments of the invention, but are not to be construed as limiting the invention in scope.

### General Experimental Procedures:

If not mentioned otherwise, chemicals are obtained in analytical grade from Merck (Darmstadt, Germany) or Sigma-Aldrich (Deisenhofen, Germany). LC-MS analyses are performed using an Agilent HP1100 (Agilent, Waldbronn, Germany) liquid chromatograph coupled with a LCT mass spectrometer (Micromass, Manchester, UK) in the positive and negative electrospray ionisation (ESI) mode, based on slight modification of a previously described method [9]. A Waters symmetry column is used as stationary phase. Mobile phase A: 0.1 % Formic acid in water, mobile phase B: 0.1 % Formic acid in acetonitrile; gradient: 0-1 min. 100 % A, from 1-6 min. to 90 % B, from 6 to 8 min to 100 % B, from 8-10 min 100 % B. LC-MS spectra are recorded in the range of molecular weights between 150 and 1.600 U. HPLC-UV/Vis analyses are carried out on a HP 1100 Series analytical HPLC system (Agilent, Waldbronn, Germany) comprising a G 1312A binary pump system, a G 1315A diode array detector, a G 1316A column compartment, a G 1322A degasser and a G 1313A autoinjector. Mobile phase: A = 0.1 % Trifluoroacetic acid in water, B = 0.1 % Trifluoroacetic acid in acetonitrile. A Macherey & Nagel (Düren, Germany) Nucleodur RP 18 column (125 x 4 mm, particle size 5 µm) serves as stationary phase. Aliquots of the samples (representing 2 - 10 µg of methanol-soluble materials, according to the concentrations of main metabolites) are analysed at 40°C with a flow of 1 ml/min in the following gradient: Linear from 0 % B to 100 % B in 20 min, thereafter isocratic conditions at 100% acetonitrile for 5 min; followed by regeneration of the column for 5 min. HPLC-UV chromatograms are recorded at 210 nm and 254 nm. Diode array detection (DAD) is employed to record HPLC-UV/Vis spectra in the range of 190 - 600 nm. The HP ChemStation software allows for an automated search for calibrated standard compounds in crude extracts. Preparative HPLC is performed at room temperature on a preparative HPLC system (Gilson Abimed, Ratingen, Germany), comprising Gilson Unipoint software, 306 binary pump system, 204 fraction collector, 155 UV-Vis detector, 806 manometric module, and 811C dynamic mixer, using different gradients and stationary phases as described below. NMR spectra are recorded on a Bruker DMX500, operating at 500.13 MHz proton frequency. All spectra are measured in DMSO-d₆ solution at 302 K. The solvent peak is used as internal reference for both proton and carbon chemical shifts (δ_{H}: 2.50, δ_{C}: 39.5).

### Example 1: Purification of Schizandrol A (1), Gomisin N (2) and Schizandrin C (3):

1 kg of dried *Schisandra chinensis* fruits (Galke, Gittelde/Harz, Germany) are extracted for 30 minutes with 1800 ml 95 % Ethanol by sonification at room temperature and finally hackled using an ultra-turrax (Janke & Kunkel, Staufen, Germany) for 10 minutes. After vacuum-filtration the remaining fruit material is extracted a second time with 700 ml 95 % Ethanol for 30 minutes. The solvent of the combined extracts is evaporated under reduced pressure. Reextraction of the remaining water phase (approximately 250 ml) is performed with 250 ml ethyl acetate for 3 times. The pooled ethyl actate extracts are evaporated under reduced pressure to dryness resulting in an extract called crude extract hereinafter.

For further enrichment of pure compounds, the first purification step of the crude extract is performed on a Nucleodur 100-5 C18ec column (Macherey & Nagel, Düren, Germany) (250 x 20 mm) using a linear water (Solvent A) / acetonitrile (Solvent B) gradient (both solvents contains 0,1 % Trifluoroacetic acid) starting with 20 % acetonitril ramped to 100 % acetonitril in 50 minutes followed by an isocratic phase for 30 minutes. With a flow rate of 20 ml/min the fractions are collected each minute. Using a UV/VIS-155 detector (Gilson, Langenfeld, Germany) the signals are detected at 210 nm and 254 nm. The fractions of crude Schizandrol A (retention time 28-33 minutes), Gomisin N (retention time 42-46 min) and Schizandrol C (retention time 45-50 minutes) are collected and purified under the same conditions with a variation of the gradient.

Schizandrol A: The linear gradient runs from 20 % B up to 70 % B in 50 minutes and continues in linear up to 100 % B within additional 10 minutes. The desired product elutes at a retention time (Rₜ) of 35-40 minutes and yield is 505 mg. The spectroscopic data comply with reported data (see Chizhov OS, Tetrahedron Letters 1961, 20, 730-734 and Ikeya Y, Taguchi H, Yosioka I, Kobayashi H; "The constituents of Schizandra chinensis Baill. I. Isolation and structure determination of five new lignans, gomisin A, B, C, F and G, and the absolute structure of schizandrin". Chem Pharm Bull (Tokyo). 1979 27(6):1383-94).

Gomisin N: The linear gradient of the second HPLC-purification runs from 60 % B to 80 % B in 30 minutes and up to 100 % B in additional 5 minutes. At a retention time of 25-27 minutes the desired product elutes in a yield of 180 mg. The spectroscopic data comply with reported data (see lkeya Y, Taguchi H, Yosioka I, Kobayashi H; "The constituents of Schizandra chinensis Baill. I. Isolation and structure determination of five new lignans, gomisin A, B, C, F and G, and the absolute structure of schizandrin". Chem Pharm Bull (Tokyo). 1979 27(6):1383-94).

Schizandrol C: Continuing the above chromatography Schizandrol C elutes (retention time of 28-32 minutes) in a yield of 65 mg. The spectroscopic data comply with reported data (see Schneiders, GE, Stevenson, R, "Structure and synthesis of (±) wuweizisu C", J. Org. Chem. 1981 46: 2969-70).

### Some Analytical Data of Schizandrol A (1) obtained:

Schizandrol A (formula (1) in Example 2) is detected by HPLC-UV and LC-MS using the methods described in General Experimental Procedures. The characteristics in analytical HPLC systems are summarized in table 1. These characteristics also serve to identify the examples by analytical HPLC in crude extracts and intermediate fractions obtained during extraction, downstream processing and chromatography.

**Table 1:**

| **Compound** | **Rₜ (HPLC-UV-Vis) [min]** | **Rₜ (LC-MS) [min]** | **Detected peak m/z (pos. ESI)** | **Detected peak m/z (neg. ESI)** |
|---|---|---|---|---|
| 1 | 12.72-12.88 | 6.25-6.50 | 415 (M+H-H₂O)⁺ 433 (M+H)⁺ | 477 (M-H+formic acid)⁻ |

¹H NMR (DMSO-*d₆*, 500 MHz): δ 0.69 (d, *J* = 7.4 Hz, 3H, 9'-H₃), 1.11 (s, 3H, 9-H₃), 1.67 (m, 1 H, 8'-H), 2.27 (dd, *J* = 13.7, 6.9 Hz, 1 H, 7'-Hₐ), 2.28 (d, *J* = 13.2 Hz, 1 H, 7-Hₐ), 2.36 (d, *J* = 13.2 Hz, 1H, 7-H_{b}), 2.70 (dd, *J* = 14.0, 1.7 Hz, 1H, 7'-H_{b}), 3.38, 3.39 (s, 3H, 3-OMe, 3'-OMe), 3.71, 3.72 (s, 3H, 4-OMe, 4'-OMe), 3.79 (s, 3H, 5'-OMe), 3.80 (s, 3H, 5-OMe), 6.65 (s, 1 H, 6'-H), 6.72 (s, 1H, 6-H).

### Some Analytical Data of Gomisin N (2) obtained:

Gomisin N (formula (2) in Example 2) is detected by HPLC-UV and LC-MS using the methods described in General Experimental Procedures. Their characteristics in analytical HPLC systems are summarized in table 2. These characteristics also serve to identify the examples by analytical HPLC in crude extracts and intermediate fractions obtained during extraction, downstream processing and chromatography.

**Table 2:**

| **Compound** | **Rₜ (HPLC-UV-Vis) [min]** | **Rₜ (LC-MS) [min]** | **Detected peak m/z (pos. ESI)** | **Detected peak m/z (neg. ESI)** |
|---|---|---|---|---|
| **2** | 17.27-17.41 | 7.55-7.69 | 401 (M+H)⁺ | No lonisation |

**Table 2a: NMR in DMSO-d₆, 500 MHz**

| **Atom** | **¹³C** | **¹H** | ***J* [Hz]** | **Int** | **COSY** | **HMBC** |
|---|---|---|---|---|---|---|
| 1 | 134.0 | - | - | - | - | - |
| 1' | 137.7 | - | - | - | - | - |
| 2 | 123.2 | - | - | - | - | - |
| 2' | 121.6 | - | - | - | - | - |
| 3 | 151.4 | - | - | - | - | - |
| 3' | 141.0 | - | - | - | - | - |
| 3-OMe | 60.0 | 3.39 | s | 3 | - | 3 |
| 3'-OMe | 58.8 | 3.68 | s | 3 | - | 3' |
| 4 | 140.0 | - | - | - | - | - |
| 4' | 134.7 | - | - | - | - | - |
| 4-OMe | 60.3 | 3.72 | s | 3 | - | 4 |
| 5 | 151.9 | - | - | - | - | - |
| 5' | 148.7 | - | - | - | - | - |
| 5-OMe | 55.5 | 3.79 | s | 3 | - | 5 |
| 6 | 110.6 | 6.66 | s | 1 | - | 1, 2, 4, 5, 7 |
| 6' | 102.8 | 6.56 | s | 1 | - | 2', 4', 5', 7', (3') |
| 7 | 38.2 | 2.29 2.55 | dd, 13.5, 1.4 dd, 13.5, 7.7 | 2 | 8 | 1, 2, 6, 8, 9, 8' |
| 7' | 34.7 | 2.20 | m | 2 | 8' | 1', 2', 6', 8', 9', 8 |
| 8 | 32.8 | 1.82 | m | 1 | 7,9 | 1, 7, 9, ', 8' |
| 8' | 40.3 | 1.66 | m | 1 | 7', 9' | 1', 9', 8, 9 |
| 9 | 12.3 | 0.65 | d, 7.1 | 3 | 8 | 7, 8, 8' |
| 9' | 21.0 | 0.92 | d, 7.1 | 3 | 8' | 7', 8', 8 |
| 10' | 100.5 | 5.97 5.99 | s s | 2 | - | 4', 5' |

### Some Analytical Data of Schizandrin C (3) obtained:

Schizandrin C (formula (3) in Example 2) is detected by HPLC-UV and LC-MS using the methods described in General Experimental Procedures. Their characteristics in analytical HPLC systems are summarized in table 3.

**Table 3:**

| **Compound** | **Rₜ (HPLC-UV-Vis) [min]** | **Rₜ (LC-MS) [min]** | **Detected peak m/z (pos. ESI)** | **Detected peak m/z (neg. ESI)** |
|---|---|---|---|---|
| 3 | 17.58-17.76 | 7.61-7.78 | 385 (M+H)⁺ | No lonisation |

¹H NMR (DMSO-*d₆*, 500 MHz): δ 0.65 (d, *J* = 7.1 Hz, 3H, 9-H₃), 0.92 (d, *J* = 7.1 Hz, 3H, 9'-H₃), 1.65 (m, 1 H, 8'-H), 1.80 (m, 1 H, 8-H), 1.97 (d, *J* = 12.8 Hz, 1 H, 7'-Hₐ), 2.05 (dd, *J* = 12.8, 9.3 Hz, 1H, 7'-H_{b}), 2.25 (d, *J* = 13.1 Hz, 1H, 7-Hₐ), 2.52 (dd, *J* = 13.1, 7.4 Hz, 1H, 7-H_{b}), 3.70, 3.72 (s, 3H, 3-OMe, 3'-OMe), 6.57 (s, 2 x 1H, 6-H, 6'-H).

### Example 2: Further enriched Extract from Schisandra chinensis

A chromatographic enrichment step of the crude extract (see Example 1) is performed on a Nucleodur 100-5 C18ec column (Macherey & Nagel, Düren, Germany) (130 x 40 mm) using a linear water (Solvent A) / acetonitrile (Solvent B) gradient (both solvents contain 0,1 % trifluoroacetic acid) starting with 30 % acetonitrile ramped to 100 % acetonitril in 60 minutes followed by an isocratic phase for 20 minutes. With a flow rate of 20 ml/min the fractions are collected each minute. Using a UV/VIS-155 detector (Gilson, Langenfeld, Germany) the signals are detected at 210 nm and 254 nm. The fractions containing Deoxyschizandrol A, Gomisin N and Schizandrin C (retention time 32-62 min) are collected and pooled. This process allows the directed enrichment of LXR active Lignans and downgrade of LXR inactive compounds.

The peaks using various detection systems in HPLC are shown in Fig. 1. The numbers of the peaks therein correspond to the compounds given in the following table 4:

**Table 4: Peaks in HPLC in Fig. 1, molmass [g/mol]**

| | Rt [min] | measured | calculated | | assigned ion |
|---|---|---|---|---|---|
| 1 | 6,41 | 432.93 | 432.21 | Schizandrol A* | M+H |
| | | 415,40 | | | M+H-H2O |
| | | 449,86 | | | M+NH4 |
| | | 881,86 | | | 2M+NH4 |
| 1a | 7,06 | 531,22 | | | M+H |
| | | 548,12 | | | M+NH4 |
| | | 1077,89 | | | 2M+NH4 |
| 1b | 7,37 | 389,15 | | | M+H |
| | | 406,19 | | | M+NH4 |
| | | 793,70 | | | 2M+NH4 |
| 2 | 7.61 | 399.21 | | | M+H-H2O |
| | | 417,25 | 416,46 | Gomisin A | M+H |
| | | 434,10 | | | M+NH4 |
| | | 849,60 | | | 2M+NH4 |
| 2a | 8,85 | 483,47 | | | M+H-H2O |
| | | 501,08 | | | M+NH4 |
| | | 518,04 | | | M+H |
| | | 1017,68 | | | 2M+NH4 |
| 3 | 9,34 | 501,03 | 500,58 | Angeloylgomisin H | M+H |
| | | 483,38 | | | M+H-H2O |
| | | 518,30 | | | M+NH4 |
| | | 1017,16 | | | 2M+NH4 |
| 3a | 9,93 | 547,98 | | | M+NH4 |
| | | 1077,57 | | | 2M+NH4 |
| 3b | 10,10 | 554,05 | | | M+NH4 |
| | | 1089,51 | | | 2M+NH4 |
| 4 | 10,69 | 515,17 | 514,58 | 7-Tigloyl-gomisin P | |
| | | 532,04 | | | |
| | | 1045,06 | | | |
| 4a | 10,85 | 515,26 | | | M+H-H2O |
| | | | 536,20 | Gomisin C* | |
| | | 554,08 | | | M+NH4 |
| | | 1089,68 | | | 2M+NH4 |
| 5 | 11,03 | 532,02 | | | M+NH4 |
| | | | 514.22 | Gomisin B* | |
| | | 1045,73 | | | 2M+NH4 |
| 5a | 11,55 | 403,31 | | | M+H |
| | | 420,07 | | | M+NH4 |
| | | 821,97 | | | 2M+NH4 |
| 6 | 13,66 | 417,25 | 416.22 | Desoxyschizandrol A | M+H |
| | | 433,98 | | | M+NH4 |
| | | 840,26 | | | 2M+NH4 |
| 7 | 14,63 | 401,19 | | | M+H |
| | | 817,23 | | | 2M+NH4 |
| 8 | 14,88 | 401,24 | 400.19 | Gomisin N* | M+H |
| | | 418,00 | | | M+NH4 |
| | | 817,50 | | | 2M+NH4 |
| 8a | 15,57 | 385,30 | 384.15 | Schizandrin C* | M+H |
| | | 401,16 | | | M+NH4 |
| | | 785,54 | | | 2M+NH4 |

Other signals (between 11 and 13 min and at 14 min, marked with a horizontal bar in fig 1.) are referring to compounds which do not show typical UV spectra of dibenzo[a,c]cyclo-octadienes. Compounds marked with an asterisk are further verified after isolation by their NMR-spectra. The retention times are identical with a deviation of smaller than 0,1 min, the mass spectra comply.

Gomisin G and Gomisin C are regio isomers with respect to the position of the methylen bridged ether group. The structure of Gomisin C is checked via its HMBC-NMR spectrum.

### HPLC conditions:

Agilent HP1100 apparatus (Agilent Technologies, Inc., Santa Clara, CA USA
Oven: Agilent G1316A
Column: Symetrie C18-150mm, 3,5 µm
AutoSampler: Agilent G1329A
Injection Volume (µl) - 2.00

| | | |
|---|---|---|
| Eluent: | | |
| A: water + 0,1 % formic acid | | 60% |
| B: Acetonitrile + 0,1 % formic acid | | 40% |
| Flow (mL/min) | | 0.400 (const.) |
| Oven Temperature (°C) | | 40.0 |
| Time (min) | A% | B% |
| 0.00 | 60 | 40 |
| 21.00 | 0.0 | 100 |
| 25.00 | 0.0 | 100 |
| 25.10 | 60 | 40 |
| 33.00 | 60 | 40 |

The flow is splitted for analysis in two equal flows after the column, that means approx. 0,2 mL/min are infunded directly into detector 2 for mass analysis and the other part passes first detector 1 (DAD and secondly detector 3 (ELSD)
Detector 1: Diode Array Detector Agilent DAD G1315A (corresponds to UV/Vis Chrom (TLC) in Fig. 1
Detector 2: Electrospray lonisation Mass Spectrometry with micrOTOF Bruker Daltonics ser 162 (corresponds to line ESI+ Chrom. (TIC) in Fig. 1), the settings for acquisition are as shown in the following table 5:

**Table 5: Acquisition parameters**

| **Source** Type | ESI |
|---|---|
| End Plate Offset | -500 V |
| Focus | Active |
| Scan Begin | 100 m/z |
| Scan End | 2200 m/z |
| Ion Polarity | Positive |
| Capillary | -4500 V |
| Nebulizer pressure | 1.6 bar |
| Dry Gas speed | 8.0 L/min |
| Dry Heater | 220 °C |
| Dry temp | 220°C |
| Set Divert Valve | Source |
| | |

| **Ion Optics** | |
|---|---|
| Capillary Exit | 90 V |
| Skimmer 1 | 30 V |
| Hexapol 1 | 22 V |
| Skimmer 2 | 22 V |
| Hexapol 2 | 21.4 V |
| Hexapol RF | 150 V |
| Transfer Time | 76.0 µs |
| Pre Pulse Storage Time | 5.0 µs |
| Set Lens 1 Storage | 35.0 V |
| Set Lens 1 Extraction | 21.7 V |
| Set Lens 2 | 7.6 V |
| Set Lens 3 | -22.0 V |
| Set Lens 4: | 0.0 V |
| Set Lens 5 | -31.0 V |
| Detector | - 1300 V |
| | |

| **TOF** | |
|---|---|
| Corrector Fill | 45 V |
| Pulsar Pull | 399 V |
| Pulsar Push | 399 V |
| Reflector | 1300 V |
| Flight Tube | 9000 V |
| Corrector Extract | 932 V |
| Detector TOF | 1980 V |
| | |

| **Mass Calibration** | |
|---|---|
| Regression Mode | Quadratic |
| C0 | 194.6329956 |
| C1 | 403459.7812500 |
| C2 | 0.0002820 |
| | |

| **Processing** | |
|---|---|
| Summation | 19481 x |
| Guessed Noise | 200 |
| Peak Width | 5 pts |
| Average Noise | 10 |
| Guessed Average | 100 |

With these settings, it is tried to select the molecular masses of the two substances Gomisin G and benzoylgomisin Q (see *Peptides* 27, **2006,** 997-1004), that is, the spectrometer is used to search by way of a "select ion count" setting for the calculated molecular masses of these two substances and the clusters to be presumed for them. Nothing is found so that these two substances appear to be absent (at least with regard to the detection limit) from the extract.
Detector 3: ELSD detection (Evaporation Light Scattering Detection) corresponds to line ELSD in Fig. 1
Cedex 75, Fa. Sedere, Conditions: pressure 3 bar, temperatur: 42°C, gain 9

The structural formulae of some of the compounds mentioned in Table 4 are as follows:

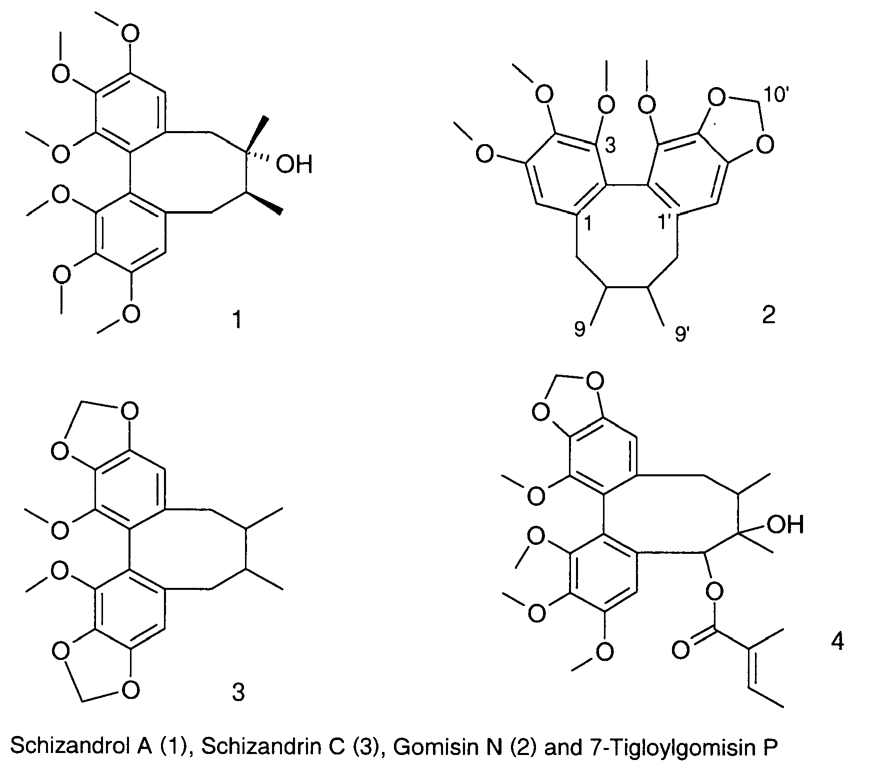

In spite of due efforts, so far it has not been possible to detect Gomisin G and benzoylgomisin Q in the extract, at least these being present in amounts so low that detection with the methods used is difficult or not possible, so that it can be assumed that these two substances are, if at all, present only in very low amounts???? and not required for the effects described in the following examples.

### Example 3: in vivo assay

The extract from Example 2 is applied as additive to food of mice to determine the weight and body fat changes as compared to controls without such addition.

Eight weeks old male mice [strain C57BU6 (Harlan Winkelmann GmbH, Borchen, Germany)] are acclimatized for two weeks by feeding with standard chow (Atromin Standardzuchtdiät 1324, Atromin Spezialfutterwerke GmbH, Lage, Germany).

### Feeding experiments:

cc: chronic balanced diet, after acclimatisation standard chow enriched with 1 mg extract per 1 g dry food.
cf: balanced/high fat diet: after acclimatisation standard chow enriched with 1 mg extract per 1 g dry food and 12 % Palmin (Peter Kölln KGaA, Elmshorn).

Mice are weighed weekly (accuracy ±0.1 g) before analyzing body composition under isoflurane (2-chloro-2-(difluoromethoxy)-1,1,1-trifluoro-ethane) anaesthesia with a DEXA scanner (PIXlmus2 scanner, software version 1.46.007, GE Medical Systems, Madison, Wisconsin, USA). The head of the animals is excluded from the measurement as recommended by the manufacturer. The GE Lunar Piximus2 DEXA scanner gives accurate information on differences in body composition in mice (see Nagy, TR and Clair, A-L. Precision and accuracy of dual-energy X-ray absorptiometry for determining in vivo body composition in mice. Obesity Res. 2000; 8:392-398; Brommage Am J Physiol Endocrinol Metab 285: E454-E459, 2003. Validation and calibration of DEXA body composition in mice; Sarah L. Johnston, Wendy L. Peacock, Lynn M. Bell, Michel Lonchampt, and John R. Speakman, Obesity Research 2005 13 (9), 1558). One scanning procedure takes up to five minutes per individual and provides data on fat mass, lean mass, bone mineral content and bone mineral density. The method is validated against Soxhlet (SOX) fat extraction in mice and a strong correlation (r 2 > 0.95) between fatDEXA and fatSOX is determined (published information).

Statistical analyses can be done by Kruskal-Wallis H-Test calculated by SigmaStat (Jandel Scientific, San Rafael, California, USA).

The results are shown in table 6 and Fig. 2

**Table 6: Weight and body fat after different treatment regimens**

| | week | feeding | weight | weight loss abs | weight loss % | body fat % |
|---|---|---|---|---|---|---|
| control | 0 | cc | 25,409 | 0 | 0 | 8,34 |
| control | 1 | cc | 24,491 | -0,918 | -3,61 | - |
| control | 2 | cc | 24,437 | -0,972 | -3,83 | 10,14 |
| extract | 0 | cc | 26,254 | 0 | 0 | 10,13 |
| extract | 1 | cc | 24,084 | -2,17 | -8,27 | - |
| extract | 2 | cc | 23,575 | -2,679 | -10,53 | 9,46 |
| control | 0 | cf | 25,415 | 0 | 0 | 9,37 |
| control | 1 | cf | 25,739 | 0,324 | 1,27 | - |
| control | 2 | cf | 25,936 | 0,521 | 2,05 | 9,61 |
| extract | 0 | cf | 25,62 | 0 | 0 | 10,07 |
| extract | 1 | cf | 25,40 | -0,21 | -0,82 | - |
| extract | 2 | cf | 25,10 | -0,52 | -2,02 | 9,99 |

| | | | | | | |
|---|---|---|---|---|---|---|
| n.d. = not determined. | | | | | | |

It can be seen that especially in the case of chronically balanced diet a weight loss can be observed if mice are given extract and that in these mice the body fat decreases in contrast to the control group, while in the case of a balanced high fat diet the controls gain weight, in contrast to the mice treated with extract which show a weight decrease.

### Example 4: Blood Brain barrier penetration

Using the web-based computer program PreADME (that allows to calculate parameters relating to adsorption, distribution, metabolism and excretion, that is pharmacodynamic parameters), http://preadmet.bmdrc.org/preadmet/index.php, the blood brain barrier permeability for compounds found above is calculated.

According to M. Ajay, G.W. Bemis and M.A. Murcko, "Designing libraries with CNS Activity", J. Med. Chem. 1999, 42, 4942-4951, compounds can be classified according to the following table with regard to their activity in CNS:

**Table 7: Classification of compounds for their blood brain barrier penetrability**

| Classification | BB ratio (C_{brain}/C_{blood}) | log BB |
|---|---|---|
| CNS - Active compounds | more than 1.0 | more than 0 |
| CNS - Inactive compounds | less than 1.0 | less than 0 |

Using this approach, for several compounds it can be shown that many of the lignane compounds found in Schisandra can be expected to rather not pass the blood brain barrier (see table 8). This may be an indication that effects on the central nervous system (e.g. on NPFF) may not play a major role in their anti-obesity effects.

**Table 8: Blood brain barrier: Prediction tool PreADME, ratio concentration brain to blood**

| Compound | BB ratio |
|---|---|
| Gomisin B | 0,0333594 |
| Gomisin C | 0,024993 |
| Desoxyschizandrin | 0,11369 |
| Gomisin N | 0.0826716 |
| Schizandrol A | 0.043378 |
| Angeloylgomisin H | 0.052546 |
| 7-Tigloylgomisin P | 0.0333594 |
| Gomisin A | 0.0342632 |

### Example 5: in vitro assay with LXR (liver X receptor)

### Assay description:

The LXR assay is configured using time-resolved fluorescence resonance energy transfer technology (HTR-FRET) (see Albers M, Blume B, Schlueter T, Wright MB, Kober I, Kremoser C, Deuschle U, Koegl M; "A novel principle for partial agonism of liver X receptor ligands. Competitive recruitment of activators and repressors". J. Biol. Chem. 2006 24;281 (8) 4920-30; and Chin J, Adams AD, Bouffard A, Green A, Lacson RG, Smith T, Fischer PA, Menke JG, Sparrow CP, Mitnaul LJ; "Miniaturization of cell-based beta-lactamase-dependent FRET assays to ultra-high throughput formats to identify agonists of human liver X receptors". Assay Drug Dev. Technol. 2003 1(6):777-87).

In the presence of an agonist, a fusion protein of glutathione S-transferase (GST) and LXR ligand binding domain (GST-LXR LBD) associates with a biotin-labeled nuclear receptor coactivator (b-SRC1). A fluorescent signal is detected in the presence of Eu-labeled anti GST antibodies and streptavidin-labeled allophycocyanin (APC). FRET is made possible by agonist-dependent close interaction of the test proteins.

Compounds which act as agonists at the LXR ligand binding domain are detected by an increase in time-resolved fluorescence. LXR-FRET is performed as described by Albers et al. (loc. cit.).

FRET-active compounds are assayed in a FRET 12-point dose response experiment to determine the EC50 - values of the compounds mentioned in the following table 9:

**Table 9:**

| Compound | EC50 |
|---|---|
| Schizandrin C | 5.6 µM |
| Gomisin N | 10.6 µM |
| Desoxyschizandrol A | >10 µM |

Thus, these experiments show that compounds from the dibenzo[a,c]cyclooctadiene class are inhibitors or liver X receptor activation. While it is not intended to be bound by this theory, this may be a possible explanation for a mechanism against obesity and for lowering blood cholesterol in animals, such as humans.

## Claims

1. A compound of the formula I, 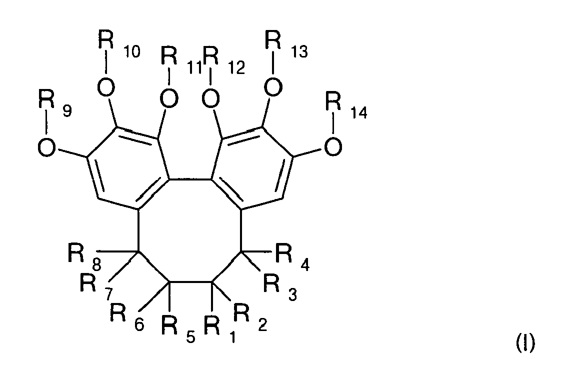 wherein
R1, R2, R3, R4, R6, R7 and R8 independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more ring heteroatoms, a substituted or unsubstituted aryl group with 6 to 18 ring atoms which can comprise one or more ring heteroatoms, a substituted or unsubstituted C₆-C₁₈-aryl-C₁-C₁₂-alkyl group which can comprise one or more heteroatoms, a substituted or unsubstituted aryloxy group wherein aryl has 6 to 18 ring atoms and can comprise one or more ring heteroatoms, a (-C(O)-Rₐ) group, a (-C(S)-Rₐ) group, a (-OC(O)-Rₐ) group, a (-OC(S)-Rₐ) group, a (-OC(O)-ORₐ) group, a (-(CH₂)ₙ-CRₐ=CRₐR_{b}) group, a (-O-(CH₂)ₙ-CRₐ=CRₐR_{b}) group or a (-OC(O)-(CH₂)ₙ-CRₐ=CRₐR_{b}) group wherein n is zero or an integer from 1 to 12 and
Rₐ and R_{b} independently from each other are selected from a group A, consisting of hydrogen, a straight-chain or branched-chain alkyl group having 1 to 12 carbon atoms, a cycloalkyl group having 3 to 10 carbon atoms and optionally comprising one or more ring heteroatoms, and a substituted or unsubstituted aryl group with 6 to 18 ring atoms which can comprise one or more ring heteroatoms, and a substituted or unsubstituted C₆-C₁₈-aryl-C₁-C₁₂-alkyl group which can comprise one or more heteroatoms; where R_{b} can further be selected from (CH₂-CH₂-CRₐ=CRₐRₐ) with Rₐ as defined above,
R1 and R5 independently from each other represent a (CH₂Rₐ) group, a (CH₂ORₐ) group, a (-C (O)-Rₐ) group, a (-C(S)-Rₐ) group, a (-COORₐ) group or a (-C(O)NRₐ) group with Rₐ as defined above;
or R3 and R4 when taken together and/or R7 and R8 when taken together respectively stand for an oxo group (=O), thioxo group (=S), imino group (=NRₐ), cyanimino group (=NCN) or alkylidene group (=CRₐR_{b}) where Rₐ and R_{b} are as defined above, or
R3 and R4 when taken together and/or R7 and R8 when taken together, respectively, forms together with the respective spiro carbon atom of the cyclooctadiene ring a 5 to 8 membered cyclic ketal structure that is unsubstituted or substituted by one or more substituents selected from group a;
or R1 and R2 when taken together and/or R5 and R6 when taken together, respectively, together with the binding atoms form a cyclic lactone and/or lactol and/or oxirane structure of selected from those with the formulae 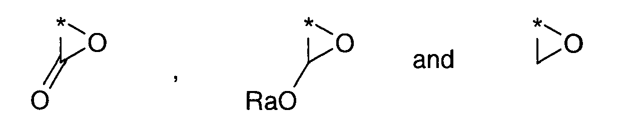 wherein the asterisk marks the carbon atom that binds R1 and R2 or R5 and R6 and Rₐ is as defined above,
or R1 and R3 or R4 when taken together and/or R5 and R7 or R8 when taken together, respectively, together with the binding atoms form a cyclic lactone and/or lactol and/or an oxetane structures selected from those with the formulae, 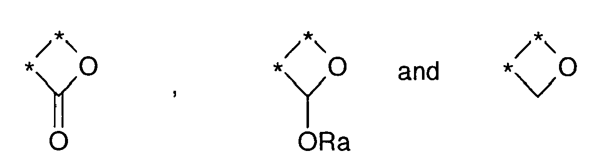 wherein the two asterisks in each formula mark the carbon atoms in formula I to which R1 and R3 or R4 and/or to which R5 and R7 or R8 are bound in formula I and Rₐ is as defined above,
or R1 and R5 when taken together and together with the binding atoms form one of the cyclic lactone, lactol, oxolane or anhydride structures of the following formulae, 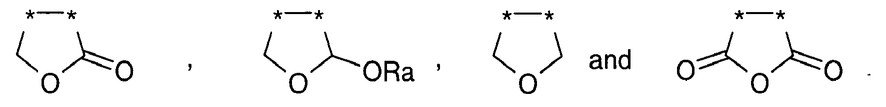 wherein the two asterisks in each formula mark the carbon atoms in formula I to which R1 and R5 are bound and Rₐ is as defined above,
or at least one of R1 and R3 or R4 when taken together and/or R5 and R7 or R8 when taken together, respectively, together with the binding atoms form a cyclic lactone and/or lactol and/or oxetane structure selected from those with the formulae, 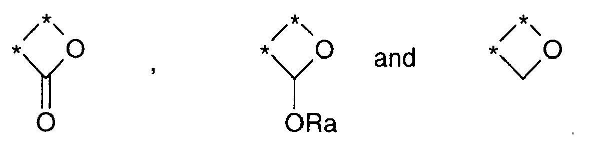 wherein the two asterisks in each formula mark the carbon atoms in formula I to which R1 and R3 or R4 or R5 and/or R7 or R8 are bound and Rₐ is as defined above,
or R1 and R7 or R8 when taken together and/or R5 and R3 or R4 when taken together, respectively, together with the binding carbon atoms and the carbon atom between them in formula I form a cyclic lactone and/or lactol and/or oxolane structure selected from those with the formulae, 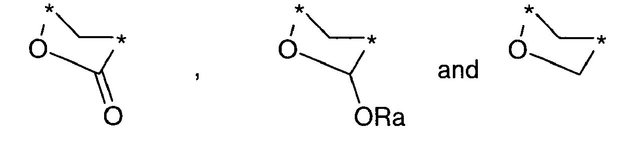 wherein the two asterisks in each formula mark the carbon atoms in formula I to which R1 and R7 or R8 and/or R5 and R3 or R4 are bound and Rₐ is as defined above,
or R1 and R6 when taken together or R5 and R2 when taken together, respectively, together with the binding carbon atoms form a cyclic lactone and/or lactol and/or oxetane structure selected from those with the formulae, 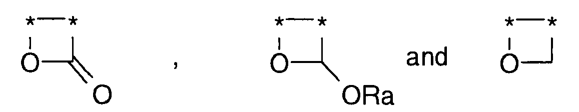 wherein the two asterisks in each formula mark the carbon atoms in formula I to which R1 and R6 or R5 and R2 are bound and wherein Rₐ is as defined above,
or R3 or R4 and R7 or R8 when taken together form oxa of the formula 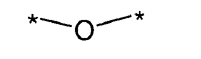 wherein the two asterisks mark the carbon atoms in formula I to which R3 or R4 and R7 or R8 are bound,
or R2 and R3 or R4 when taken together and/or R6 and R7 or R8 when taken together, respectively, together with the binding carbon atoms form a cyclic oxirane structure of the formula 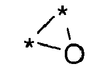 wherein the two asterisks mark the carbon atoms in formula I to which R2 and R3 or R4 and/or R6 and R7 or R8 are bound; and
R9, R10, R11, R12, R13 and R14 independently from each other represent hydrogen, a straight-chain or branched-chain alkyl group having 1 to 12 carbon atoms, a cycloalkyl group having 3 to 10 carbon atoms and optionally comprising one or more ring heteroatoms, a substituted or unsubstituted aryl group with 6 to 18 ring atoms which can comprise one or more ring heteroatoms, a substituted or unsubstituted C₆-C₁₈-aryl-C₁-C₁₂-alkyl group which can comprise one or more heteroatoms, a (-C(O)-Rₐ) group, a (-C(S)-Rₐ) group or a(-(CH₂)ₙ-CRₐ=CRₐR_{b}) group wherein n is zero or an integer from 1 to 12 and with Rₐ and R_{b} as being defined above, or
at least one of R9 and R10 when taken together, R10 and R11 when taken together, R12 and R13 when taken together and R13 and R14 when taken together, respectively, forms a straight-chain or branched-chain alkylen group having 1 to 4 carbon atoms which can comprise a heteroatom or a straight-chain or branched-chain alkenylen group having 2 to 4 carbon atoms and 1 or 2 double bonds which can comprise a heteroatom, so that together with the binding carbon atoms they form a ring;
where if one or more heteroatoms mentioned above are present they are present instead of one or more carbon atoms and are selected from the group consisting of S, N, NH, O, P and Se, preferably S, N, NH and O; with the proviso that if one or both substances with the names Gomisin G and benzoylgomisin Q are part of an extract or compound mixture useful according to the invention, at least one further compound of the formula I other than Gomisin G and benzoylgomisin Q is present, or preferably the extract or compound mixture does not comprise Gomisin G and benzoylgomisin Q,
a mixture of compounds of the formula I, and/or an extract comprising one or more compounds of the formula I, for use as active ingredient in the therapeutic - including prophylactic - treatment of an animal for the regulation of body weight and/or fat loss and/or for the management of obesity and/or for decreasing blood cholesterol; where the compound(s) of the formula I may be present in free form, in the form of a pharmaceutically and/or nutraceutically acceptable salt, in the form of tautomers, in the form of esters and/or in the form of solvates.

2. A compound of the formula I, a mixture of compounds of the formula I, and/or an extract comprising one or more compounds of the formula I, for use according to claim 1, where in the compound(s) of the formula I
R1, R2, R3, R4, R5, R6, R7 and R8 are, independently of each other, hydrogen, a straight chain or branched alkyl with 1 to 5 carbon atoms, hydroxy or (-OC(O)-(CH₂)ₙ-CRₐ=CRₐR_{b}) wherein Rₐ and R_{b} are independently of each other alkyl with 1 to 5 carbon atoms, and
R9, R10, R11, R12, R13 and R14 independently from each other represent hydrogen, a straight-chain or branched-chain alkyl group having 1 to 5 carbon atoms, a cycloalkyl group having 5 to 10 ring atoms which can comprise one or more heteroatoms, a substituted or unsubstituted aryl group with 6 to 10 ring atoms which can comprise one or more heteroatoms, a substituted or unsubstituted aralkyl group which can comprise one or more heteroatoms, a (-C(O)-Rₐ) group, a (-C(S)-Rₐ) group or a(-(CH₂)ₙ-CRₐ=CRₐR_{b}) group wherein n is zero or an integer from 1 to 12 and with Rₐ and R_{b} as being defined above, or
at least one of R9 and R10 when taken together and R13 and R14 when taken together, respectively, forms a ring containing a straight-chain or branched-chain alkylen group having 1 to 4 carbon atoms which can comprise a heteroatom or a straight-chain or branched-chain alkenylen group having 2 to 4 carbon atoms and 1 or 2 double bonds,
where the heteroatoms if present are independently selected from O, S, N and NH; and where "substituted" means that one or more, especially up to three, substituents independently selected from the group consisting of C₁-C₇-alkyl, hydroxy, C₁-C₇-alkoxy, C₁-C₇-alkanoyloxy, C₁-C₇-alkoxycarbooxy, C₁-C₇-alkanesulfonyloxy, phenyl-C₁-C₇-alkoxy, amino, N-mono- or N,N-di-(C₁-C₇-alkyl, C₁-C₇-alkanoyl, C₁-C₇-alkoxycarbonyl, C₁-C₇-alkanesulfonyl and/or phenyl-C₁-C₇-alkyl)-amino, carboxy, C₁-C₇-alkoxycarbonyl, carbamoyl, N-mono- or N,N-di-(C₁-C₇-alkyl)-carbamoyl, sulfamoyl, N-mono- or N,N-di-(C₁-C₇-alkyl)-sulfamoyl and cyano are present,
where the compound(s) of the formula I may be present in free form, in the form of a pharmaceutically and/or nutraceutically acceptable salt, in the form of tautomers, in the form of esters and/or in the form of solvates.

3. A compound of the formula I, a mixture of compounds of the formula I, and/or an extract comprising one or more compounds of the formula I, for use according to claim 1, where the compound(s) of the formula I are selected from the group consisting of those with the following names:
Schizandrol A, Gomisin B, Gomisin C, Schisandrin C, Deoxyschizandrol A, Gomisin N, Gomisin A, 7-Tigloyl-gomisin P, Angeloylgomisin H and Schizandrin C.

4. A compound of the formula I, a mixture of compounds of the formula I, and/or an extract comprising one or more compounds of the formula I, for use according to any one of claims 1 to 3, where the use is for the regulation of body weight and/or fat loss and/or for the management of obesity, especially for decreasing the body weight, more especially for decreasing the body fat,
where the compound(s) of the formula I may be present in free form, in the form of a pharmaceutically and/or nutraceutically acceptable salt, in the form of tautomers, in the form of esters and/or in the form of solvates.

5. A compound of the formula I, a mixture of compounds of the formula I, and/or an extract comprising one or more compounds of the formula I, for use according to any one of claims 1 to 3, where the use is for decreasing blood cholesterol, especially LDL cholesterol.

6. An extract, compound of the formula I or mixture of compounds according to any one of claims 1 to 6, obtained solely from plants or parts of plants from a genus selected from the group consisting of *Schisandra, Illicium, Kadsura, Steganotaenia* and *Magnolia.*

7. An extract comprising one or more compounds of the formula I as shown in any one of claims 1 to 6, preferably for use according to any one of claims 1 to 6, obtainable by extracting *Schisandra chinensis* fruits with ethanol under sonification at room temperature and finally hackling them, optionally re-extracting the remaining fruit material a second time with ethanol, combining the extracts, drying them, if desired re-extracting a remaining water phase with ethyl acetate, and drying the pooled extracts to obtain a crude extract, and further enriching the extract on a reversed phase material, eluting with a water/acetonitrile gradient, and collecting and pooling the fractions that comprise Deoxyschizandrol A, Gomisin N and Schizandrin C.

8. An extract according to claim 7, showing an HPLC pattern essentially similar to that shown in Fig. 1 according to 1, preferably 2, more preferably all three lines/methods of analysis shown there.

9. The use of a compound of the formula I, a mixture of compounds of the formula I and/or an extract as defined in any one of claims 1 to 8 for the manufacture of a pharmaceutical and/or nutraceutical preparation and/or dietary supplement and/or functional food for use as active ingredient in the therapeutic - including prophylactic - treatment of an animal and/or human for the regulation of body weight and/or fat loss and/or for the management of obesity, especially for decreasing the body weight, more especially for decreasing the body fat, and/or for decreasing blood cholesterol, especially LDL cholesterol, where the compound(s) of the formula I may be present in free form, in the form of a pharmaceutically and/or nutraceutically acceptable salt, in the form of tautomers, in the form of esters and/or in the form of solvates.

10. The use according to claim 10 for the manufacture of a pharmaceutical preparation.

11. The use according to claim 10 for the manufacture of a nutraceutical preparation.

12. The use according to claim 10 for the manufacture of a dietary supplement.

13. The use according to claim 10 for the manufacture of or as a functional food.

14. A pharmaceutical and/or nutraceutical composition and/or dietary supplement and/or functional food for use in the therapeutic - including prophylactic - treatment of an animal and/or human for the regulation of body weight and/or fat loss and/or for the management of obesity, especially for decreasing the body weight, more especially for decreasing the body fat, and/or for decreasing blood cholesterol, especially LDL cholesterol, where the active ingredient for said use is a compound of the formula I, a mixture of compounds of the formula I and/or an extract as defined in any one of claims 1 to 8, where the compound(s) of the formula I may be present in free form, in the form of a pharmaceutically and/or nutraceutically acceptable salt, in the form of tautomers, in the form of esters and/or in the form of solvates.
